# EUROPEAN PATENT APPLICATION

(11) **EP 3 845 555 A1**
(43) Date of publication of application: **07.07.2021**
(21) Application number: 20181803.6
(22) Date of filing: 23.06.2020
(51) Int. Cl.: C07K 14/50, A61Q 19/08, C12N 15/79, C12R 1/89

(54) **VECTOR FOR PRODUCING AND EXPRESSING HUMAN GROWTH FACTOR AND MICROALGAE TRANSFORMED WITH THE VECTOR**

(30) Priority: 31.12.2019 KR 20190179893; 31.12.2019 KR 20190179902; 31.12.2019 KR 20190179909
(71) Applicant: Algaeprona Inc., Gangwon-do (KR)
(72) Inventor: Shin, Bok Kyu, Gangneung-si (KR); Kwon, Byeo Ri, Gangneung-si (KR); Kim, Seul Ki, Gangneung-si (KR)
(74) Representative: Cabinet Chaillot

(57) **Abstract**

The present invention provides a vector for producing and expressing human growth factors and microalgae transformed with the vector.

## Description

### [BACKGROUND OF THE INVENTION]

### 1. Field of the Invention

The present invention relates to a vector for producing and expressing type 1 human fibroblast growth factor (hFGF1), type 2 human fibroblast growth factor (hFGF2) and type 1 human insulin-like growth factor (hIGF1), as well as microalgae transformed with the vector.

### 2. Description of the Related Art

Type 1 human fibroblast growth factor (hFGF1), type 2 human fibroblast growth factor (hFGF2) and type 1 human insulin-like growth factor (hIGF1) are functional proteins used as raw materials for wrinkle-improving cosmetics. Currently, most of such growth factors are produced and used from *E. coli.* However, *E. coli* contains endotoxin possibly implying a risk due to the same.

Accordingly, an alternative product to replace existing E. coli-produced hFGF1, hFGF2 and hIGF1 raw materials, from which the risk due to the endotoxin is substantially eliminated, is required.

### [SUMMARY OF THE INVENTION]

In view of the above problems, the inventors of the present invention have conducted research on a method capable of effectively producing hFGF1, hFGF2 and hIGF1 proteins while blocking possibility of tolerance to toxicity, leading to the present invention.

One aspect of the present invention is to provide a vector which includes a nucleotide sequence encoding type 1 human fibroblast growth factor (hFGF1) of SEQ ID NO: 2.

Another aspect of the present invention is to provide a vector which includes a nucleotide sequence encoding type 2 human fibroblast growth factor (hFGF2) of SEQ ID NO: 3.

Another aspect of the present invention is to provide a vector which includes a nucleotide sequence encoding type 1 human insulin-like growth factor (hIGF1) of SEQ ID NO: 4.

According to one aspect of the present invention, the above vector may be a vector including any one of nucleotide sequences represented by SEQ ID NO: 8 to SEQ ID NO: 10.

Another aspect of the present invention is to provide microalgae transformed with the vector.

Another aspect of the present invention is to provide a method for producing hFGF1, hFGF2 and hIGF1 proteins using the transformed microalgae.

According to one aspect of the present invention, there is provided a vector, including a growth factor expression region which includes a gene encoding hFGF1, hFGF2 or hIGF1.

According to another aspect of the present invention, the growth factor expression region may further include Hig-tag, GST-tag, GFP sequence and TEV sequence.

According to another aspect of the present invention, the growth factor expression region may be one of nucleotide sequences represented by SEQ ID NO: 2 to SEQ ID NO: 4.

According to another aspect of the present invention, the vector may be one of nucleotide sequences represented by SEQ ID NO: 8 to SEQ ID NO: 10.

According to another aspect of the present invention, there is provided microalgae transformed with the vector according to any one of the above aspects.

According to another aspect of the present invention, the microalgae may be *Phaeodactylum tricornutum.*

According to another aspect of the present invention, there is provided a cosmetic composition, including hFGF1, hFGF2 and hIGF1 proteins produced by a production method according to the above aspects.

According to another aspect of the present invention, the cosmetic composition may be a cosmetic composition for improving wrinkles.

According to another aspect of the present invention, there is provided a kit including the transformed microalgae.

The microalgae according to one aspect of the present invention may produce hFGF1, hFGF2 and hIGF1 proteins without tolerance to toxicity.

The hFGF1, hFGF2 and hIGF1 proteins produced according to one aspect of the present invention may replace such growth factor proteins produced from existing *E. coli.*

The hFGF1, hFGF2 and hIGF1 proteins produced according to one aspect of the present invention are excellent in effects of improving wrinkles.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 illustrates a vector structure according to an embodiment of the present invention, which includes: HSPA1p: HSPA1 promoter, TEV: tobacco etch virus (TEV cleavage site), 3'-UTR: 3 terminal untranslated region, FcpB: fucoxanthin/chlorophyll binding protein B promoter, shble: zeocin resistance gene;
FIG. 2 shows results of confirming gene insertion through gDNA-PCR;

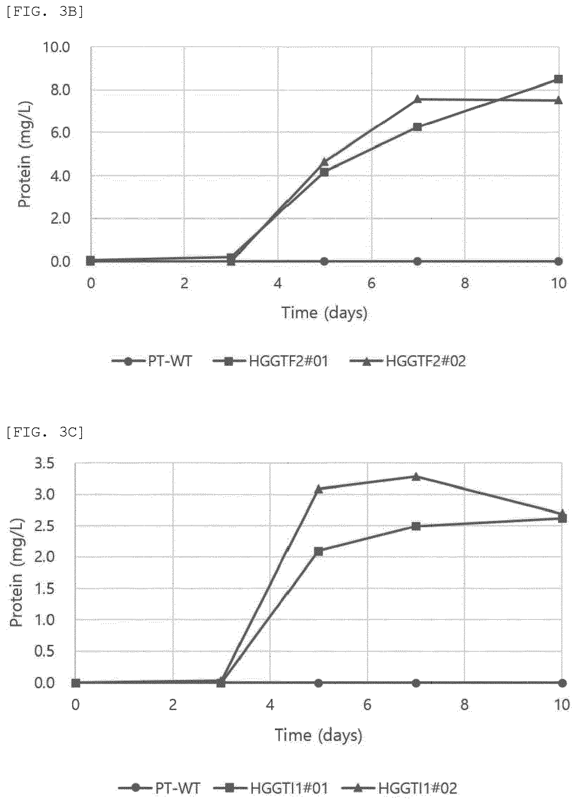

FIG. 3 shows results of confirming expression of a target protein during culturing through GFP florescence measurement;

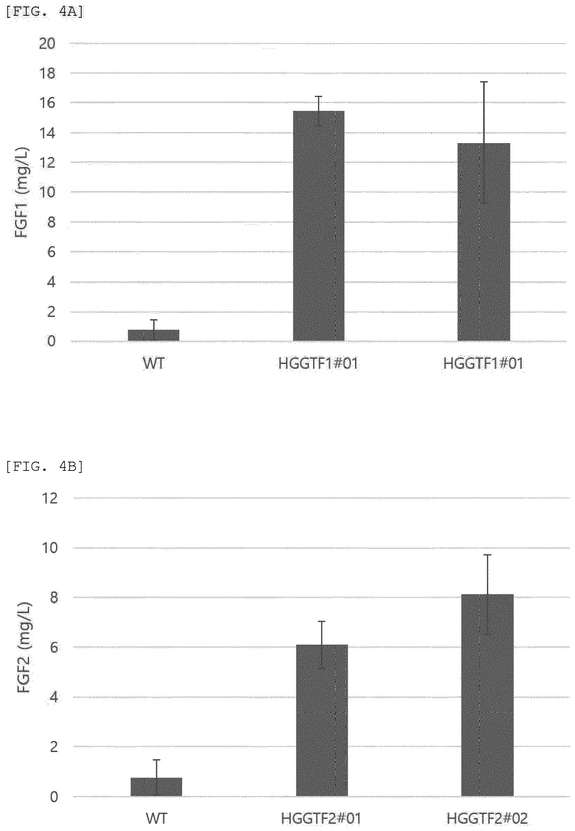

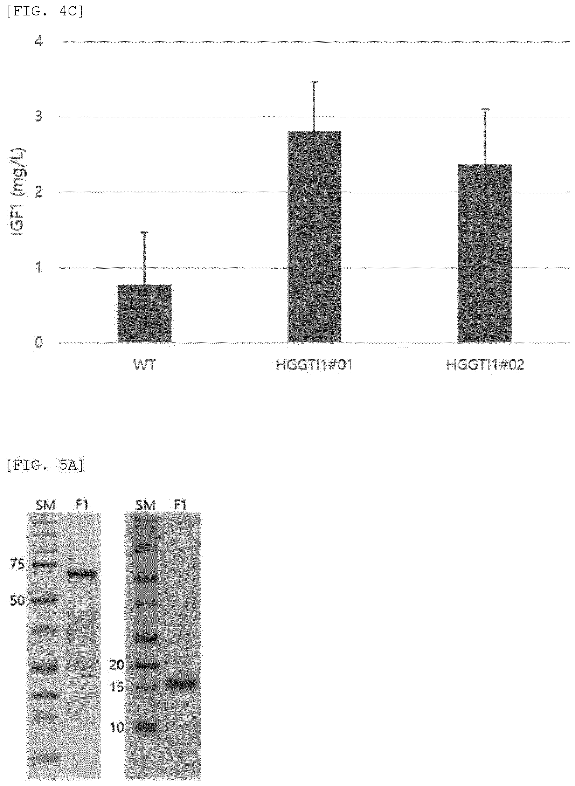

FIG. 4 shows results of confirming protein expression through ELISA;

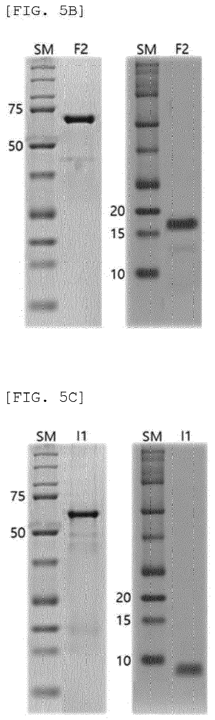

FIG. 5 shows results of target protein purification;

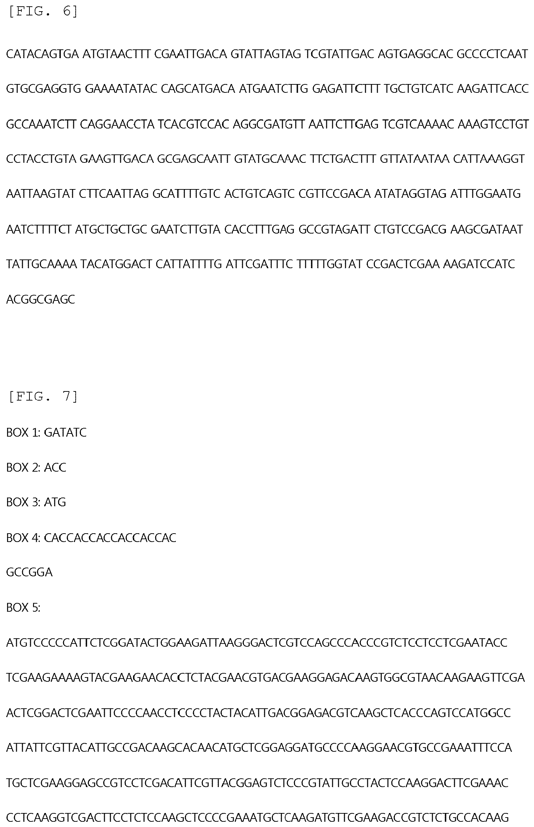

FIG. 6 illustrates a nucleotide sequence of HASP1 of SEQ ID NO: 1;

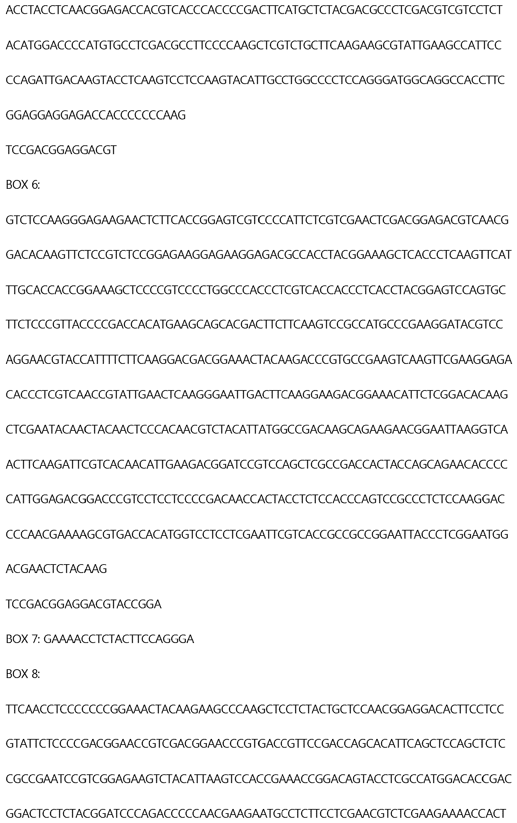

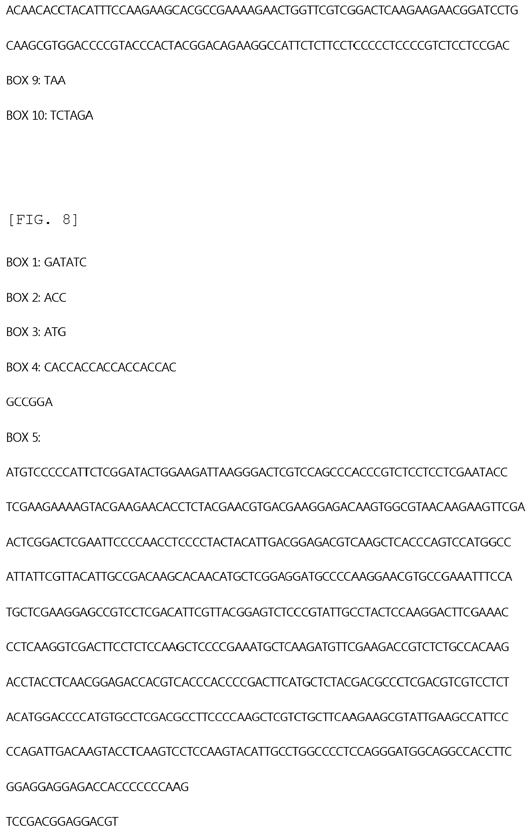

FIG. 7 illustrates a nucleotide sequence of His-GST-GFP-TEV-hFGF1 (HGGTF1) of SEQ ID NO: 2 [box 1,10: restriction enzyme site, box 8: hFGF1, box 2: kozak sequence, box 3: initiation codon, box 9: stop codon, box 4: His-tag, box 5: GST-tag, box 6: GFP, box 7: TEV site];

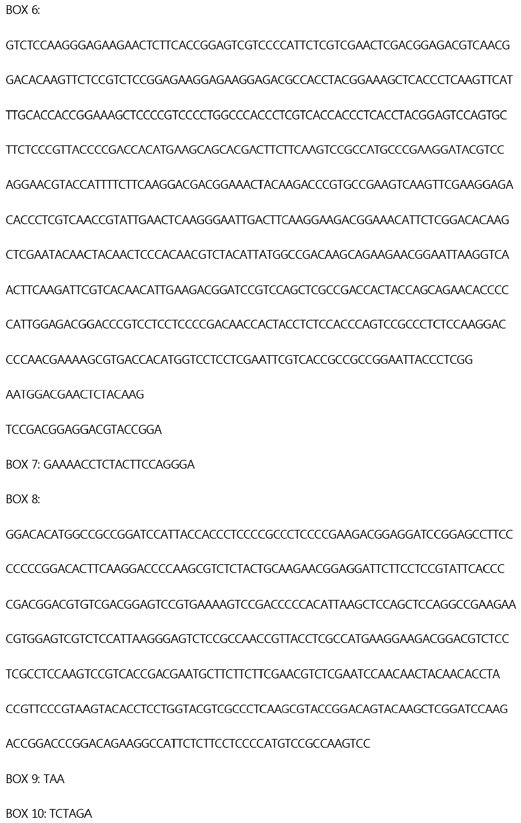

FIG. 8 illustrates a nucleotide sequence of His-GST-GFP-TEV-hFGF2 (HGGTF2) of SEQ ID NO: 3 [box 1,10: restriction enzyme site, box 8: hFGF2, box 2: kozak sequence, box 3: initiation codon, box 9: stop codon, box 4: His-tag, box 5: GST-tag, box 6: GFP, box 7: TEV site];

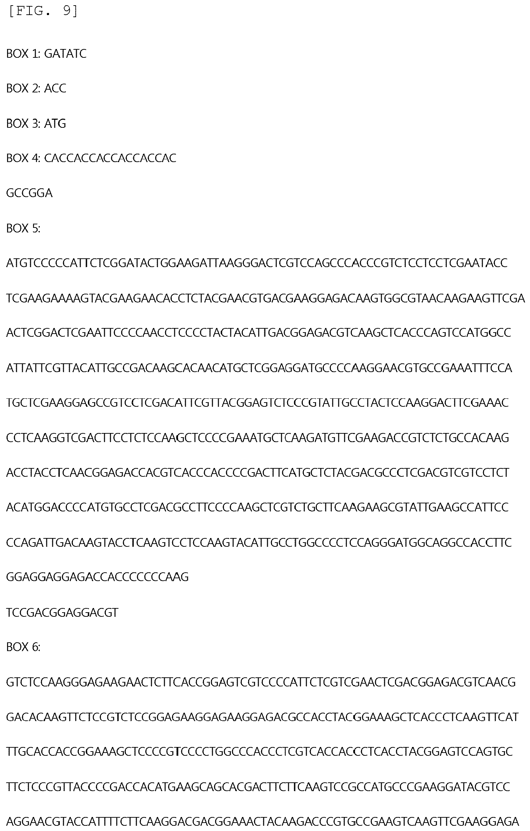

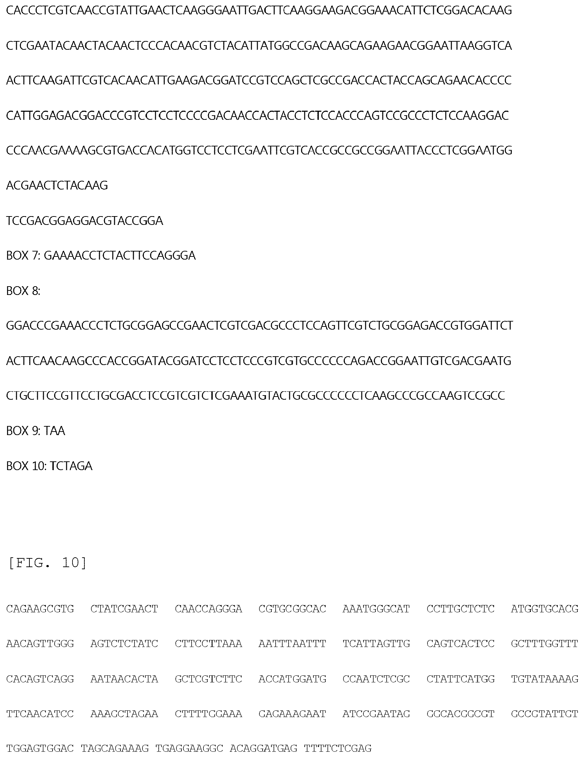

FIG. 9 illustrates a nucleotide sequence of His-GST-GFP-TEV-IGF1 (HGGT11) of SEQ ID NO: 4 [box 1,10: restriction enzyme site, box 8: hIGF1, box 2: kozak sequence, box 3: initiation codon, box 9: stop codon, box 4: His-tag, box 5: GST-tag, box 6: GFP, box 7: TEV site];
FIG. 10 illustrates a nucleotide sequence of 3'-UTR of SEQ ID NO: 5;

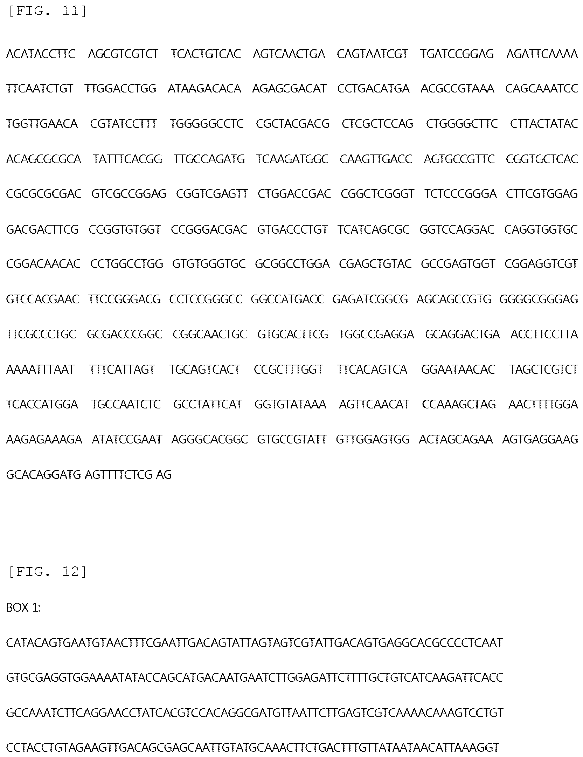

FIG. 11 illustrates a nucleotide sequence of Shble cassette of SEQ ID NO: 6;

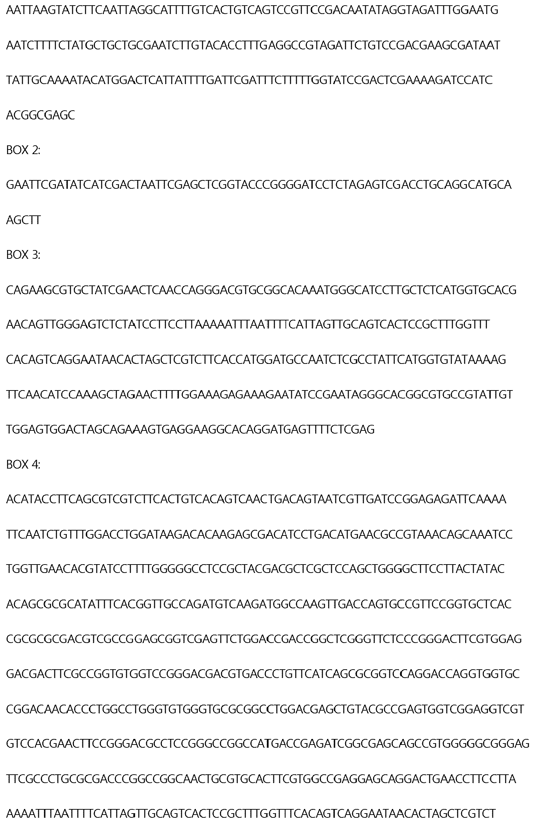

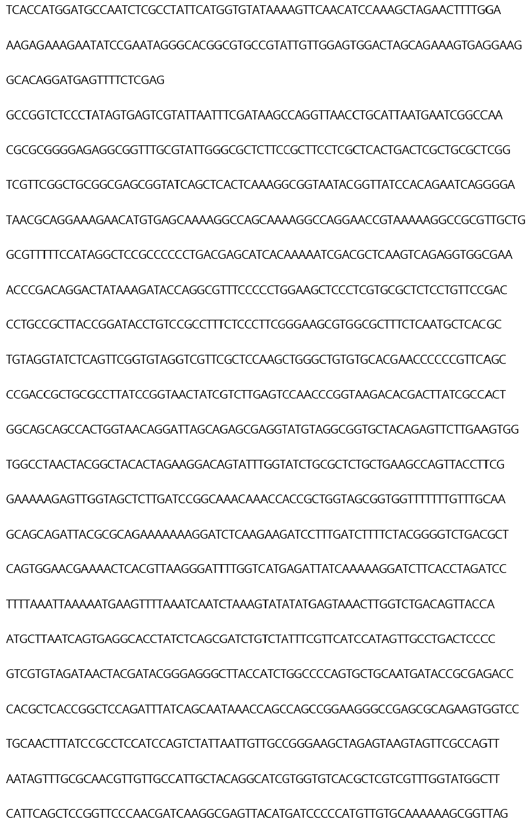

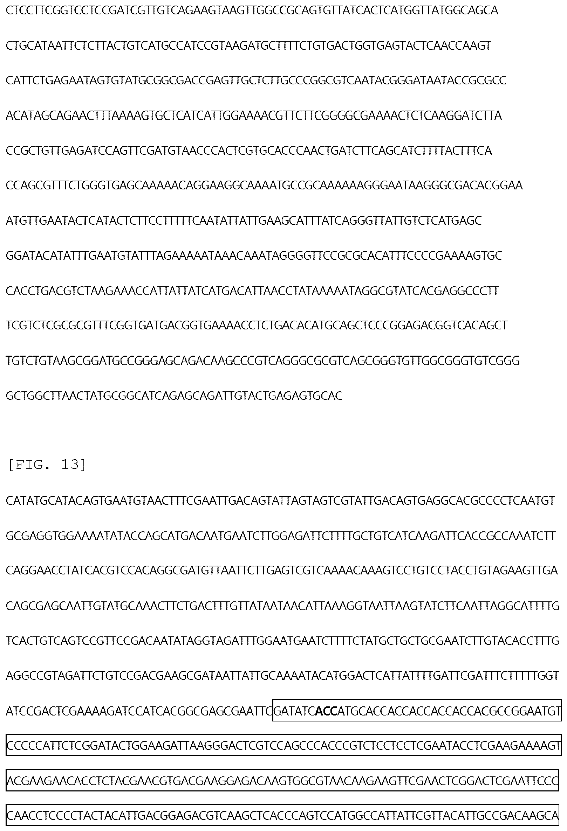

FIG. 12 illustrates a nucleotide sequence of pPha-HASPlp vector full sequence of SEQ ID NO: 7 [BOX 1: HASP1 promoter, BOX 3: 3'-UTR, BOX 4: shble cassette, BOX 2: multi-cloning site];

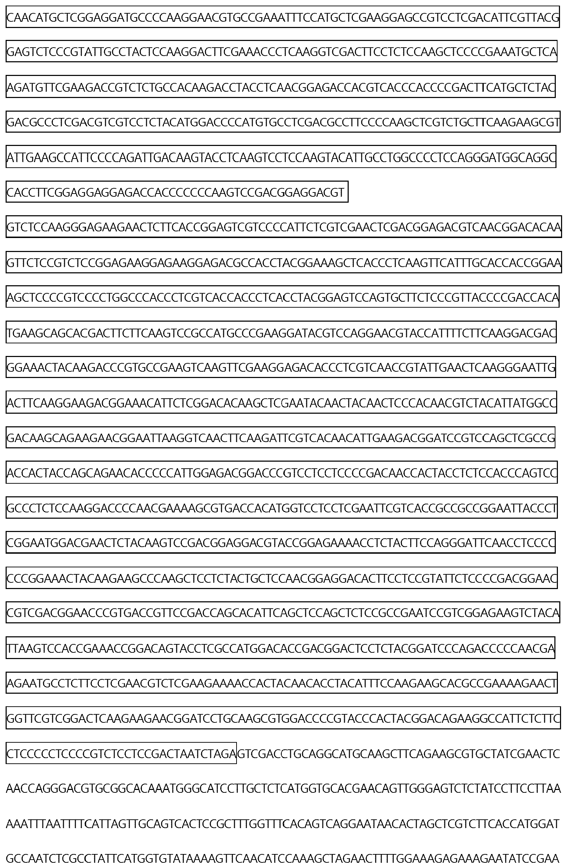

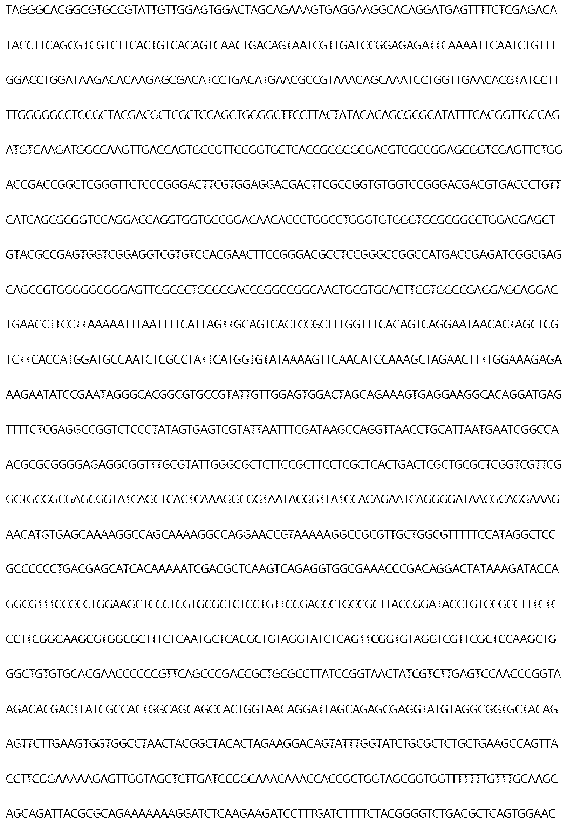

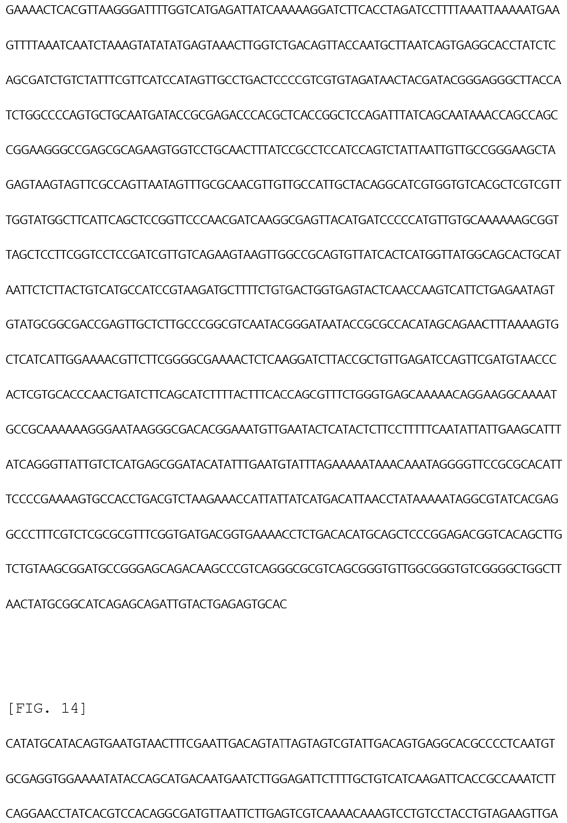

FIG. 13 illustrates a nucleotide sequence of pPha-HASP1p-HGGTF1 of SEQ ID NO: 8;

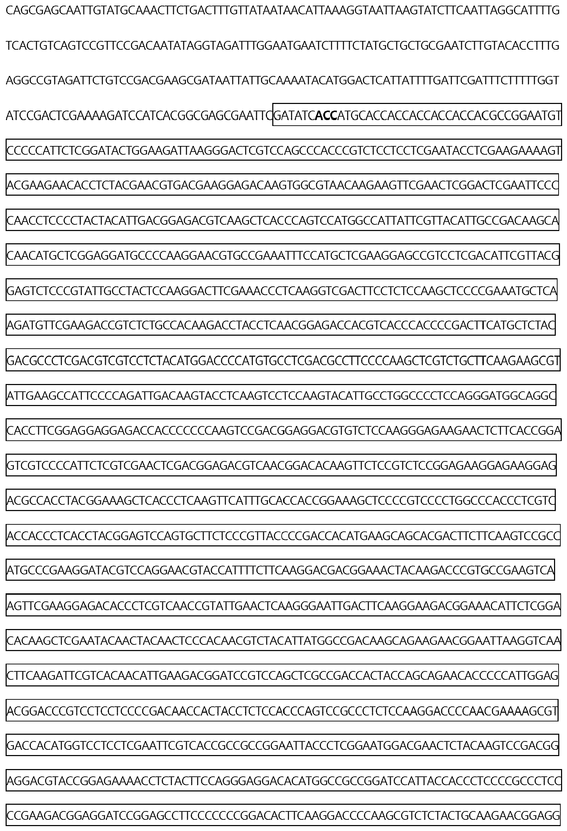

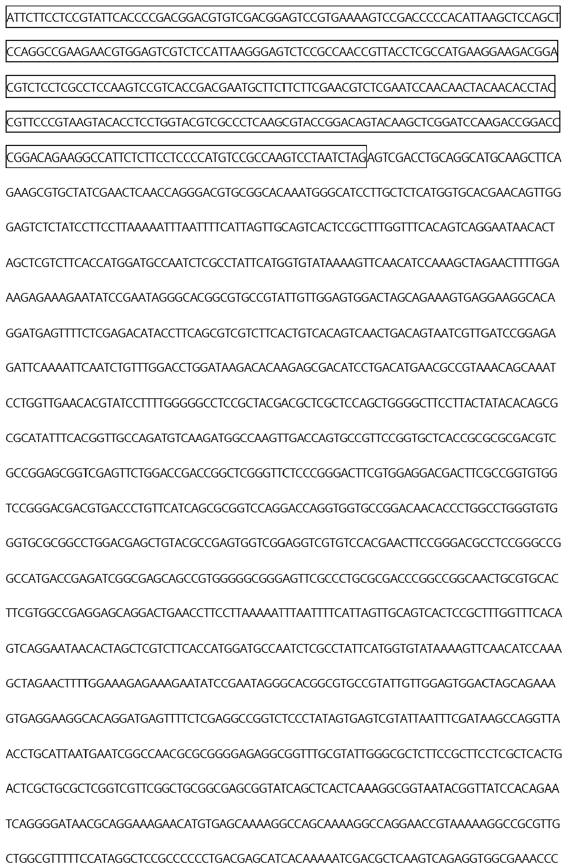

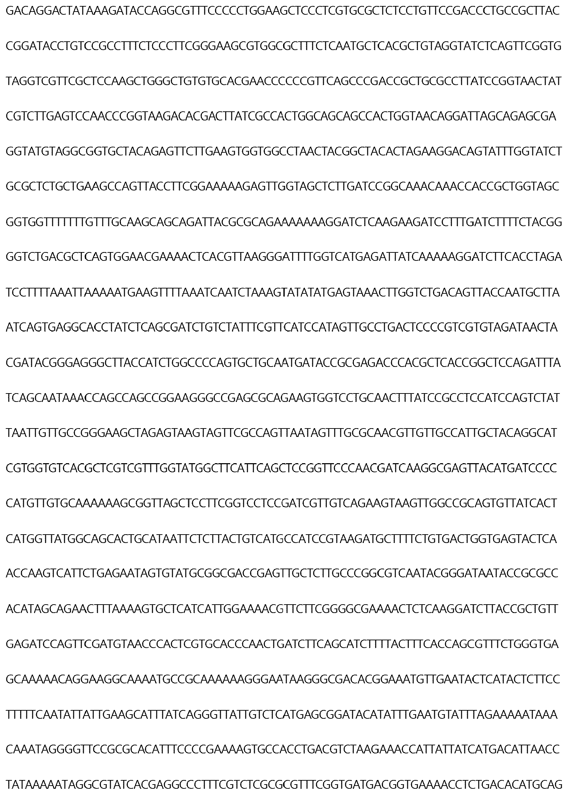

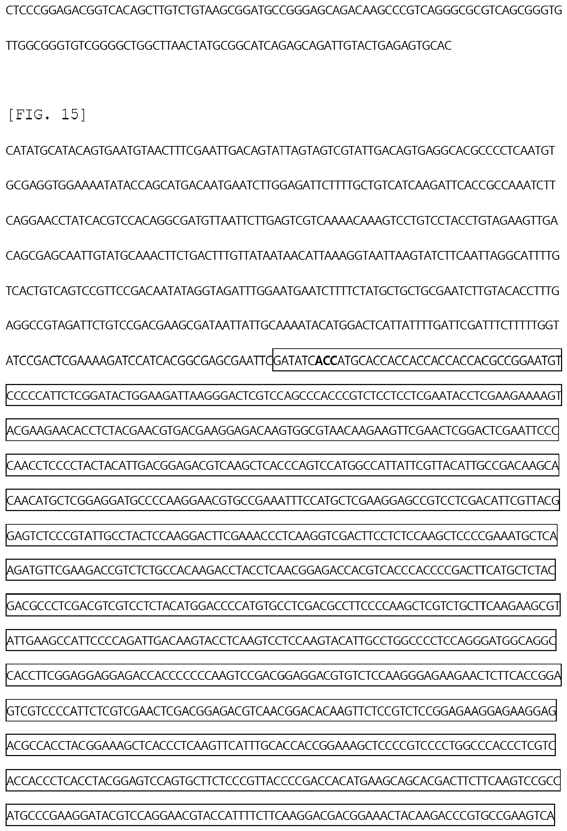

FIG. 14 illustrates a nucleotide sequence of pPha-HASP1p-HGGTF2 of SEQ ID NO: 9; and

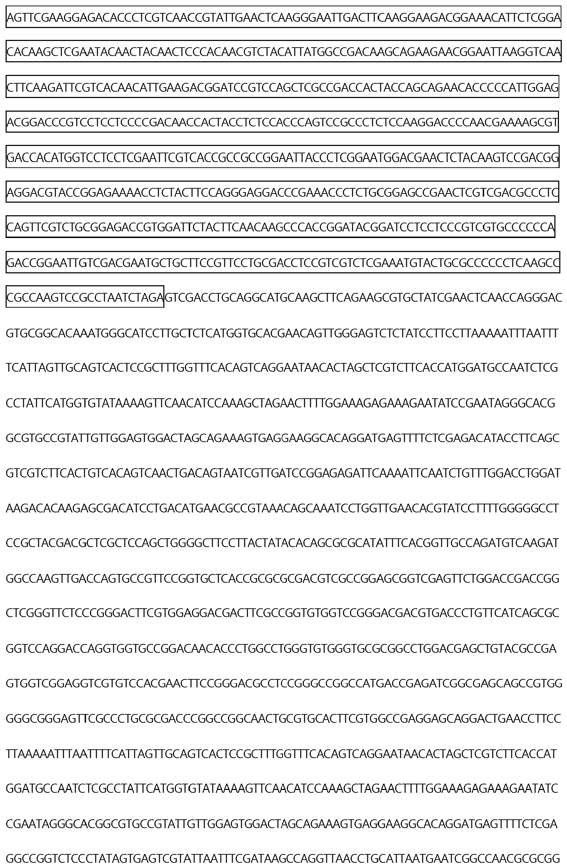

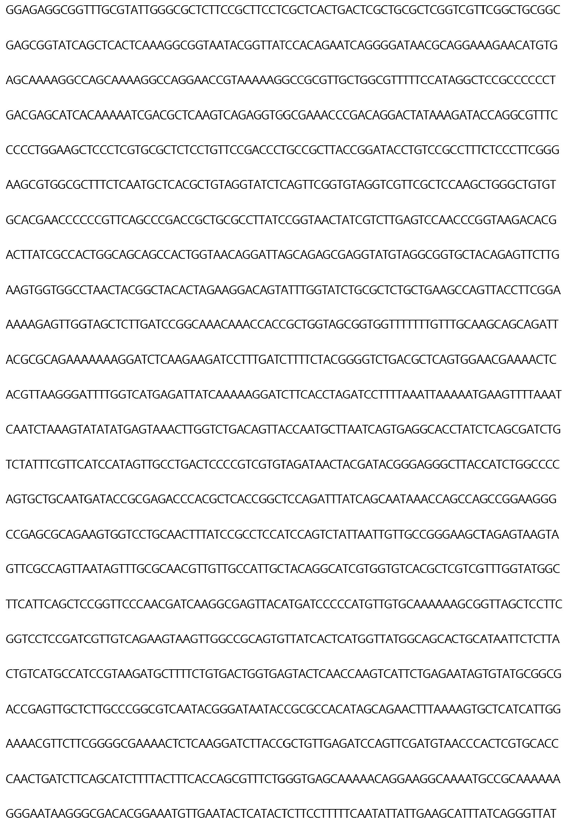

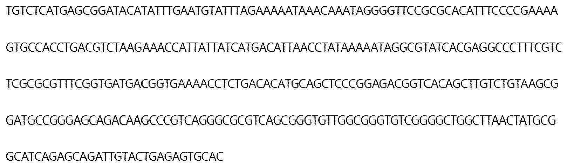

FIG. 15 illustrates a nucleotide sequence of pPha-HASP1p-HGGT11 of SEQ ID NO: 10.

### [DETAILED DESCRIPTION OF THE INVENTION]

The above and other aspects, features, and advantages of the invention will become apparent from the detailed description of the embodiments to be described in detail below in conjunction with the accompanying drawing. It should be understood that the present invention is not limited to the following embodiments and may be embodied in various different ways, and that the embodiments are given to provide complete disclosure of the invention and to provide a thorough understanding of the invention to those skilled in the art. The scope of the invention is defined only by the claims.

In description of preferred embodiments of the present invention, the publicly known functions and configurations that are judged to be able to make the purport of the present invention unnecessarily obscure will not be described in detail. Further, wordings to be described below are defined in consideration of the functions of the present invention, and may differ depending on the intentions of a user or an operator or custom. Accordingly, such wordings should be defined on the basis of the contents of the overall specification.

In the present invention, "type 1 human fibroblast growth factor (hFGF1, FGF1 or aFGF)" is a polypeptide having a molecular weight of 17 to 18 kDa, which is composed of 155 amino acids and functions as a growth factor and a signal protein.

In the present invention, "type 2 human fibroblast growth factor type (hFGF2, FGF2 or bFGF)" is a polypeptide having a molecular weight of 18 kDa, which is composed of 155 amino acids and functions as a growth factor and a signal protein.

In the present invention, "type 1 human insulin-like growth factor (hIGF1 or IGF1)" is a polypeptide having a molecular weight of about 8 kDa, which is composed of 70 amino acids.

According to one aspect of the present invention, when hFGF1, hFGF2 or hIGF1 is produced from microalgae, the risk due to endotoxin may be substantially eliminated, and the above microalgae may be used as a substitute for existing *E*. coli-produced hFGF1, hFGF2 and hIGF1 raw materials. Such microalgae-produced hFGF1, hFGF2 or hIGF1 may be useful as raw materials of cosmetics, which meet the recent cosmetic trend, that is, environment-friendly and naturalization trends.

Hereinafter, the present invention will be described in detail.

An aspect of the present invention provides a vector including: a promoter of SEQ ID NO: 1; and an expression region of type 1 human fibroblast growth factor (hFGF1), type 2 human fibroblast growth factor (hFGF2) or type 1 human insulin-like growth factor (hIGF1), which includes a gene encoding any of the above growth factors.

According to another aspect of the present invention, the growth factor expression region may be a vector further including Hig-tag, GST-tag, GFP and TEV site sequence.

According to another aspect of the present invention, the growth factor expression region may be a vector which is a nucleotide sequence represented by SEQ ID NO: 2 to SEQ ID NO: 4.

According to another aspect of the present invention, the above vector may be a vector including a nucleotide sequence represented by SEQ ID NO: 8 to SEQ ID NO: 10.

According to another aspect of the present invention, the above vector may be an encoded vector, which includes a nucleotide sequence represented by SEQ ID NO: 8 to SEQ ID NO: 10. Specifically, SEQ ID NO: 8 to SEQ ID NO: 10 may include HASP1 promoter sequence, His-tag sequence, GFP, TEV sequence, FGF1 or FGF2, IGF1 sequence, 3'-UTR sequence and Shble cassette.

The above Shble cassette may include FcpB, Shble and 3'-UTR.

Another aspect of the present invention provides microalgae transformed with the vector according to any one of the above aspects.

According to another aspect of the present invention, the microalgae may be microalgae, which is *Phaeodactylum tricornutum. Phaeodactylum tricornutum* is readily available from foreign microalgae banks. Specifically, *Phaeodactylum tricornutum* (Bohlin) may be accepted and used from a distribution organization, UTEX, with a distribution number of UTEX 646. Further, *Phaeodactylum tricornutum* (Bohlin) may be accepted and used from another distribution organization, CCAP, with a distribution number of CCAP1052/6. Furthermore, *Phaeodactylum tricornutum* may be accepted and used from another distribution organization, CCMP, with a distribution number of CCMP2559.

Another aspect of the present invention provides a method for producing hFGF1, hFGF2 and hIGF1 proteins using the transformed microalgae.

Another aspect of the present invention provides a cosmetic composition including hFGF1, hFGF2 and hIGF1 proteins produced by the above production method.

According to another aspect of the present invention, the cosmetic composition may be a cosmetic composition for improving wrinkles.

The hFGF1, hFGF2 and hIGF1 proteins included during manufacture of cosmetics including the same may be contained in an amount of 0.00001 to 10.0% by weight ('wt.%') (in dry weight), preferably, 0.001 to 1.0 wt.% based on a total amount of the composition. If the amount is less than 0.00001 wt.% (in dry weight) based on the total weight of the composition, remarkable effects of improving wrinkles could not be expected. If the amount is more than 10.0 wt.%, a significant increase in effects along an increase in the content is seldom expected.

The cosmetic composition of the present invention may include components used in a typical cosmetic composition in addition to the above-mentioned active ingredient. For example, conventional supplements such as antioxidants, stabilizers, solubilizers, vitamin, pigments and aromatic essences, as well as carriers, may be included.

The cosmetic composition of the present invention may be prepared into any of formulations generally produced in the art, and for example, may be formulated in the form of solution, suspension, emulsion, paste, gel, cream, lotion, powder, soap, surfactant-containing cleanser, oil, powdered foundation, emulsion foundation, wax foundation, spray, and the like, but it is not limited thereto. More specifically, the composition may be manufactured into any formulation such as soft lotion, nutritional lotion, nutritional cream, massage cream, essence, eye cream, cleansing cream, cleansing foam, cleansing water, pack, spray or powder.

When the formulation of the present invention is a paste, cream or gel, an animal oil, vegetable oil, wax, paraffin, starch, tragacanth, cellulose derivative, polyethylene glycol, silicone, bentonite, silica, talc or zinc oxide may be used as a carrier component.

When the formulation of the present invention is a powder or a spray, lactose, talc, silica, aluminum hydroxide, calcium silicate or polyamide powder may be used as a carrier component. In particular, in the case of a spray, a propellant such as chlorofluorohydrocarbon, propane/butane or dimethyether may be further included.

When the formulation of the present invention is a solution or an emulsion, a solvent, dissolving agent or emulsifying agent is used as a carrier component. For instance, water, ethanol, isopropanol, ethyl carbonate, ethyl acetate, benzyl alcohol, benzyl benzoate, 1,3-butyl glycol oil, glycerol aliphatic ester, polyethylene glycol or sorbitan fatty acid esters may be used.

When the formulation of the present invention is a suspension, water, a liquid diluent such as ethanol or propylene glycol, a suspension such as ethoxylated isostearyl alcohol, polyoxyethylene sorbitol ester and polyoxyethylene sorbitan ester, microcrystalline cellulose, aluminum metahydroxide, bentonite, agar or tragacanth, etc. may be used as a carrier component.

When the formulation of the present invention is a surfactant-containing cleanser, the carrier component used herein may include, for example, aliphatic alcohol sulfate, aliphatic alcohol ether sulfate, sulfosuccinic acid monoester, isethionate, imidazolinium derivative, methyltaurate, sarcosinate, fatty acid amide ether sulfate, alkylamido betaine, aliphatic alcohol, fatty acid glyceride, fatty acid diethanolamide, vegetable oil, lanolin derivative, or ethoxylated glycerol fatty acid ester.

Another aspect of the present invention provides a kit including the transformed microalgae.

Hereinafter, the present invention will be described in more detail with reference to examples. It would be obviously understood by those skilled in the art that these examples are only for illustrating the present invention and that the scope of the present invention is not construed as being limited by the same.

### [Example 1] Vector construction

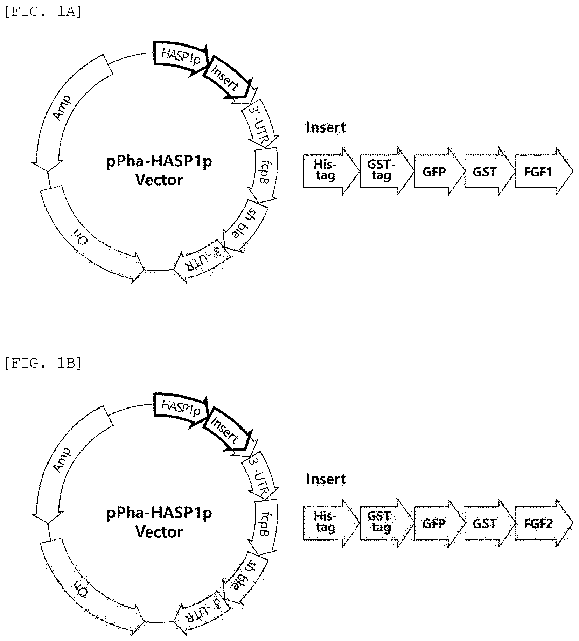

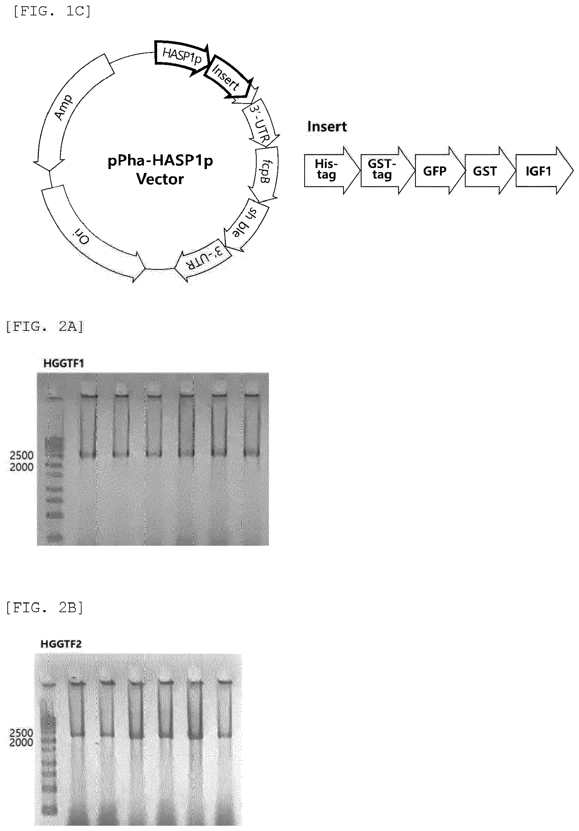

A vector composition was prepared by the following procedures.
▪ 50 mL of *P. tricornutum* culture solution incubated in F/2 medium (50% artificial seawater) was taken and centrifuged (3500 rpm, 15 minutes, 20 °C) to obtain cell pellets, followed by extracting genomic DNA (gDNA) from the same.
▪ Using the extracted gDNA as a template, DAN in HASP1 promoter site was obtained from HASP1-F and HASP1-R primers and pfu polymerase through PCR. Further, Fcp a promoter of pPha-T1 vector was substituted using Ndel and EcoRI restriction enzymes, thereby constructing pPha-HASP1p vector.
▪ hFGF1, hFGF2 and hIGF1 expression vectors containing the promoter portion were constructed as shown in FIG. 1, thereby constructing a vector capable of producing the above growth factors as target proteins.
▪ A sequence constituting the above vector was shown in Table 1 below.

**[TABLE 1]**

| | |
|---|---|
| SEQ ID NO: 1 | HASP1 promoter |
| SEQ ID NO: 2 | His-GST-GFP-TEV-hFGFl (HGTF1) |
| SEQ ID NO: 3 | His-GST-GFP-TEV-hFGF2 (HGTF2) |
| SEQ ID NO: 4 | His-GST-GFP-TEV-hIGF1 (HGTI1) |
| SEQ ID NO: 5 | 3'-UTR |
| SEQ ID NO: 6 | Shble cassette (containing FcpB, Shble, 3'-UTR) |
| SEQ ID NO: 7 | pPha-HASP1p vector |
| SEQ ID NO: 8 | pPha-HASP1p-hGTF1 |
| SEQ ID NO: 9 | pPha-HASP1p-hGTF2 |
| SEQ ID NO: 10 | pPha-HASP1p-hGTI1 |

**[TABLE 2]**

| # | Name | Sequence | Restrict ion enzyme | Annotation |
|---|---|---|---|---|
| 1 | HASP1-F | CATATGCATACAGTGAATGTAACTTTC G | NdeI | pPha-HASP1; PCR and Genomic-DNA PCR |
| 2 | HASP1-R | GAATTCGCTCGCCGTGATGGATCTTTT C | EcoRI | pPha-HASP1p; PCR |
| 3 | pPha-Up-NdeI-F | GTGTCGGGGCTGGCTTAAC | | Genomic DNA PCR |
| 4 | pPha-Down-MCS-R | GCCGCACGTCCCTGGTTG | | Genomic DNA PCR |

### [Example 2] Microalgae transformation

Microalgae transformation was conducted as follows.
▪ After the vectors for producing target proteins in Example 1 were transformed in *E.coli* (DH5a (NEB® 5-alpha Competent E.coli), the transformed *E.coli* was cultured in LB (100 µg/mL ampicillin) medium.
▪ Target vectors were extracted from the cultured *E.coli* by Midiprep method, and then, high purity vector (with a concentration of 1.0 µg/µL) required for *P*. *tricornutum* transformation was obtained by phenol/chloroform gene purification and ethanol purification.
▪ After coating gold nanoparticles with the vector, the target vector was transferred to *P. tricornutum* via particle bombardment, thereby completing transformation.
▪ The transformant was subjected to screening on F/2 agar medium containing zeocin (100 µg/mL).

The transformants obtained according to the above procedures are designated as *P. tricornutum* (PT-HASP1p-HGGTF1), *P. tricornutum* (PT-HASP1p-HGGTF2) and *P. tricornutum* (PT-HASP1p-HGGTI1), which were deposited with KCTC 14080BP, 14081BP and 14082BP, respectively, to Korean Collection for Type Culture (KCTC) on December 18, 2019.

### [Experimental Example 1] Confirmation of expression of hFGF1, hFGF2 and hIGF1

Insertion of genes encoding hFGF1, hFGF2 and hIGF1 into *P. tricornutum* transformants selected in Example 3 was confirmed through gDNA-PCR. Further, expression of a target protein was confirmed by ELISA. Specifically, the confirmation was performed in Experimental Examples 1 and 2 below.

### [Experimental Example 2] Confirmation of gene insertion through gDNA-PCR

### (1) Experimental method

▪ The hFGF1, hFGF2 and hIGF1 expression transformant colonies in Example 2 were confirmed.
▪ Insertion of *P. tricornutum* into genomic DNA was confirmed through gDNA-PCR.
▪ Each transformant colony was inoculated in 200 µL of F/2 (50% artificial seawater) medium and cultured for 5 days, followed by centrifugation (3500 rpm, 15 minutes, 20 °C), and then a supernatant was removed.
▪ 10 minutes after addition of 10 µL dissolution buffer (1% Nonidet P-40), the solution was used for PCR.
▪ Using pPha-Up-NdeI-F, pPha-Down-MCS-R primer and taq polymerase, PCR was conducted for confirmation of gene insertion.

### (2) Experimental result

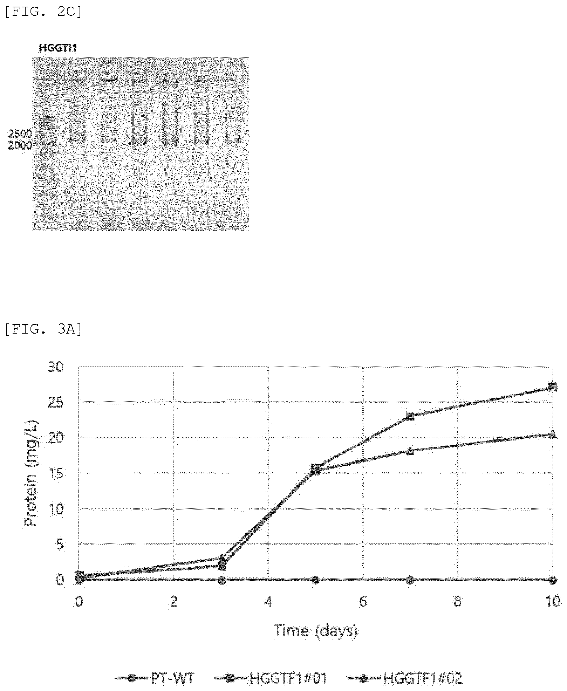

▪ FIG. 2 shows the results of DNA electrophoresis. Results of gDNA-PCR of hFGF1 (FIG. 2A), hFGF2 (FIG. 2B) and hIGF1 (FIG. 2C) are observed. As a result of the observation, it could be confirmed that target gene bands were present at similar sites.
▪ As a control, a blank transformant having an empty vector inserted without insertion of a target gene was used. In the control, the target gene band was not observed.

### [Experimental Example 3] Confirmation of expression of hFGF1, hFGF2 and hIGF1 through GFP fluoroimetry

### (1) Experimental method

▪ An expression level of target protein in the microalgae was determined through GFP combined with target proteins, that is, hFGF1, hFGF2 and hIGF1 N-terminal.
▪ 200 µL of microalgae solution was dispensed in a 96 well plate, followed by measurement using a microplate reader (Biotek, Synergy HTX) (excitation: 485/20, emission: 528/20).
▪ An expression level of protein in the microalgae was determined by the same method through a standard curve.

### (2) Experimental results

▪ It was confirmed that the target protein was successfully expressed in the transformed microalgae.
▪ For hFGF1 and hFGF2, the expression became to appear on 5^{th} day of culture, and the expression levels after 7^{th} day of culture were measured as 23.0 mg/L and 7.57 mg/L, respectively.
▪ For hIGF1, the expression appeared on 5^{th} day of culture, and the expression level measured till the 5^{th} day was 31 mg/L.

### [Experimental Example 4] Confirmation of expression of hFGF1, hFGF2 and hIGF1 through ELISA

### (1) Experimental method

▪ Contents of hFGF1, hFGF2 and hIGF1 were determined by ELISA kit for enzymes (abchem, ab219636, ab99979, ab211651).
▪ After each transformant was cultured in F/2 (50% artificial seawater) for 10 days, 20 mL of the cultured product was taken and centrifuged to obtain cell pellets, followed by adding 5 mL of dissolution buffer (50 mL Tris-HCl, 150 mM NaCl, 1 mM PMSF, 0.1% Tween 20, 1 mM EDTA, 1% Nonidet P40) .
▪ Microalgae cells were pulverized by a sonicator (10% power, working for 10 seconds - resting and cooling for 50 seconds, 10 times).
▪ After removing debris through centrifugation (10,000 rpm, 20 minutes, 4 °C), the supernatant was used as an ELISA sample.

### (2) Experimental result

▪ For hFGF1, the expression level of 15.4 mg/L was confirmed.
▪ For hFGF2, the expression level of 8.1 mg/L or more was confirmed.
▪ For hIGF1, the expression level of 2.8 mg/L or more was confirmed.

### [Experimental Example 5] Purification of hFGF1, hFGF2 and hIGF1 and SDS-PAGE confirmation

### (1) Experimental method

▪ After each transformant was cultured in F/2 (50% artificial seawater) for 10 days, 2 L of the cultured product was taken and centrifuged to obtain cell pellets, followed by adding 20 mL of dissolution buffer (50 mL Tris-HCl, 150 mM NaCl, 1 mM PMSF, 0.1% Tween 20, 1 mM EDTA, 1% Nonidet P40).
▪ Microalgae cells were pulverized by a sonicator (30% power, working for 10 seconds - resting and cooling for 50 seconds, 10 times).
▪ After removing debris through centrifugation (10,000 rpm, 20 minutes, 4 °C), the supernatant was used for purification.
▪ First adsorption chromatography was conducted using Ni-resin 5 mL (His-trap 5 mL, GE healthcare).
▪ A result of the chromatography was confirmed through 12% Tricine-SDS-PAGE.
▪ After removing salt through PD10 column (Hi-desalt 5 mL, GE healthcare), the product was treated with TEV protease at a concentration of 5 to 10 µg/mL, followed by reaction at 4 °C for 9 hours.
▪ Second adsorption chromatography was conducted using Ni-resin 5 mL (His-trap 5 mL, GE healthcare), so as to remove TEV and ensure a target protein with removal of a tag sequence.
▪ A result of the chromatography was confirmed through 15% Tricine-SDS-PAGE.

### (2) Experimental result

▪ HGGTF1 (His-GST-GFP-TEV-FGF1, 71.1 kDa) protein band was observed.
▪ HGGTF2 (His-GST-GFP-TEV-FGF1, 72.7 kDa) protein band was observed.
▪ HGGTI1 (His-GST-GFP-TEV-IGFl, 62.9 kDa) protein band was observed.
▪ Final hFGF1 (15.8 kDa) protein band was observed.
▪ Final hFGF2 (17.4 kDa) protein band was observed.
▪ Final hIGF1 (7.76 kDa) protein band was observed.

### [Experimental Example 6] Test for wrinkle improving effect

Generally, wrinkle-improving effects may be determined by collagen biosynthesis ability, collagenase decomposition inhibitory ability and clinical tests to human.

Human normal fibroblasts were inoculated on 6-well microplate containing DMEM medium (2×10⁵ cells/well) and then cultured in a 5% CO₂ incubator at 37 °C for 24 hours. After 24 hours, the medium was removed from each well and treated with samples at different concentrations, followed by culturing again for 24 hours. After 24 hours, the cell medium was collected and used to prepare a sample.

Collagen synthesis level was determined by measuring an amount of procollagen type I C-peptide (PICP) in the cell medium using a collagen measurement kit, that is, Procollagen type I C-peptide EIA kit (M101) (Dakara, Kyoto, Japan), followed by analysis of the result.

As a method for determining activity of collagenase to decompose collagen, an antibody to collagenase was used. The collagenase activity was measured using type I collagenase assay kit (Amersham Biosciences, RPN2629). Further, absorbance was measured by ELISA reader. The measured standard value was indicated in the form of mean±standard deviation (SD). Further, t-test significance was verified by SPSS/PC+ program, and results thereof are shown in Table 3 below.

**[TABLE 3]**

| Sample | Increase of collagen synthesis (%) | Collagenase inhibitory rate (%) |
|---|---|---|
| hFGF1 (10 µg/ml) | 72 | 40 |
| hFGF2 (10 µg/ml) | 75 | 41 |
| hIGF1(10 µg/ml) | 73 | 40 |

As shown in Table 3, it could be seen that collagen synthesis was increased while inhibiting collagenase activity by addition of purified hFGF1, hFGF2 and hIGF1 in Experimental Example 5. Accordingly, it could be understood that hFGF1, hFGF2 and hIGF1 of the present invention had wrinkle-improving efficacy.

The above description is merely illustrative of the technical idea of the present invention, and those skilled art to which the present invention pertains will appreciate that various modifications and variations are possible without departing from the essential characteristics of the present invention. Therefore, the embodiments disclosed in the present invention are intended to describe the technical spirit of the present invention, and are not intended to limit the same, as well as the scope of the technical spirit of the present invention is not limited to these embodiments. It should be understood that the protective scope of the present invention is interpreted by the claims below, and all technical ideas within the equivalent range are included in the scope of the present invention.

## Claims

1. A vector, comprising a growth factor expression region which includes a gene encoding a human growth factor.

2. The vector according to claim 1, wherein the gene encoding the human growth factor includes one selected from the group consisting of genes encoding type 1 human fibroblast growth factor (hFGF1), type 2 human fibroblast growth factor (hFGF2) and human insulin growth factor (hIGF1).

3. The vector according to claim 1, wherein the growth factor expression region further includes Hig-tag, GST-tag, GFP sequence and TEV sequence.

4. The vector according to claim 1, wherein the growth factor expression region is one selected from nucleotide sequences represented by SEQ ID NO: 2, SEQ ID NO: 3 and SEQ ID NO: 4.

5. The vector according to claim 1, wherein the vector is one selected from nucleotide sequences represented by SEQ ID NO: 8, SEQ ID NO: 9 and SEQ ID NO: 10.

6. Microalgae transformed with the vector according to claim 1.

7. A method for producing one selected from hGF1, hFGF2 and hIGF1 by culturing the microalgae according to claim 6.

8. A cosmetic composition, comprising one selected from hFGF1, hFGF2 and hIGF1 produced by the method according to claim 7 as an active ingredient.

9. The cosmetic composition according to claim 8, wherein the cosmetic composition is used for improving wrinkles.


<110>     ALGAEPRONA INC.

<120>     VECTOR FOR PRODUCING AND EXPRESSING HUMAN GROWTH FACTOR AND
MICROALGAE TRANSFORMED WITH THE VECTOR

<130>     SP1100

<160>     10

<170>     KoPatentIn 3.0

<210>     1
<211>     499
<212>     DNA
<213>     Phaeodactylum tricornutum

<220>
<221>     promoter
<222>     (1)..(499)
<223>     HASP1 promoter


<400>     1
catacagtga atgtaacttt cgaattgaca gtattagtag tcgtattgac agtgaggcac          60

gcccctcaat gtgcgaggtg gaaaatatac cagcatgaca atgaatcttg gagattcttt         120

tgctgtcatc aagattcacc gccaaatctt caggaaccta tcacgtccac aggcgatgtt         180

aattcttgag tcgtcaaaac aaagtcctgt cctacctgta gaagttgaca gcgagcaatt         240

gtatgcaaac ttctgacttt gttataataa cattaaaggt aattaagtat cttcaattag         300

gcattttgtc actgtcagtc cgttccgaca atataggtag atttggaatg aatcttttct         360

atgctgctgc gaatcttgta cacctttgag gccgtagatt ctgtccgacg aagcgataat         420

tattgcaaaa tacatggact cattattttg attcgatttc tttttggtat ccgactcgaa         480

aagatccatc acggcgagc                                                      499


<210>     2
<211>     1890
<212>     DNA
<213>     Artificial Sequence

<220>
<223>     His-GST-GFP-TEV-hFGF1 (HGGTF1)


<400>     2
gatatcacca tgcaccacca ccaccaccac gccggaatgt cccccattct cggatactgg          60

aagattaagg gactcgtcca gcccacccgt ctcctcctcg aatacctcga agaaaagtac         120

gaagaacacc tctacgaacg tgacgaagga gacaagtggc gtaacaagaa gttcgaactc         180

ggactcgaat tccccaacct cccctactac attgacggag acgtcaagct cacccagtcc         240

atggccatta ttcgttacat tgccgacaag cacaacatgc tcggaggatg ccccaaggaa         300

cgtgccgaaa tttccatgct cgaaggagcc gtcctcgaca ttcgttacgg agtctcccgt         360

attgcctact ccaaggactt cgaaaccctc aaggtcgact tcctctccaa gctccccgaa 420

atgctcaaga tgttcgaaga ccgtctctgc cacaagacct acctcaacgg agaccacgtc 480

acccaccccg acttcatgct ctacgacgcc ctcgacgtcg tcctctacat ggaccccatg 540

tgcctcgacg ccttccccaa gctcgtctgc ttcaagaagc gtattgaagc cattccccag 600

attgacaagt acctcaagtc ctccaagtac attgcctggc ccctccaggg atggcaggcc 660

accttcggag gaggagacca cccccccaag tccgacggag gacgtgtctc caagggagaa 720

gaactcttca ccggagtcgt ccccattctc gtcgaactcg acggagacgt caacggacac 780

aagttctccg tctccggaga aggagaagga gacgccacct acggaaagct caccctcaag 840

ttcatttgca ccaccggaaa gctccccgtc ccctggccca ccctcgtcac caccctcacc 900

tacggagtcc agtgcttctc ccgttacccc gaccacatga agcagcacga cttcttcaag 960

tccgccatgc ccgaaggata cgtccaggaa cgtaccattt tcttcaagga cgacggaaac 1020

tacaagaccc gtgccgaagt caagttcgaa ggagacaccc tcgtcaaccg tattgaactc 1080

aagggaattg acttcaagga agacggaaac attctcggac acaagctcga atacaactac 1140

aactcccaca acgtctacat tatggccgac aagcagaaga acggaattaa ggtcaacttc 1200

aagattcgtc acaacattga agacggatcc gtccagctcg ccgaccacta ccagcagaac 1260

acccccattg gagacggacc cgtcctcctc cccgacaacc actacctctc cacccagtcc 1320

gccctctcca aggaccccaa cgaaaagcgt gaccacatgg tcctcctcga attcgtcacc 1380

gccgccggaa ttaccctcgg aatggacgaa ctctacaagt ccgacggagg acgtaccgga 1440

gaaaacctct acttccaggg attcaacctc ccccccggaa actacaagaa gcccaagctc 1500

ctctactgct ccaacggagg acacttcctc cgtattctcc ccgacggaac cgtcgacgga 1560

acccgtgacc gttccgacca gcacattcag ctccagctct ccgccgaatc cgtcggagaa 1620

gtctacatta agtccaccga aaccggacag tacctcgcca tggacaccga cggactcctc 1680

tacggatccc agacccccaa cgaagaatgc ctcttcctcg aacgtctcga agaaaaccac 1740

tacaacacct acatttccaa gaagcacgcc gaaaagaact ggttcgtcgg actcaagaag 1800

aacggatcct gcaagcgtgg accccgtacc cactacggac agaaggccat tctcttcctc 1860

cccctccccg tctcctccga ctaatctaga 1890

<210> 3
<211> 1941
<212> DNA
<213> Artificial Sequence

<220>
<223> His-GST-GFP-TEV-hFGF2 (HGGTF2)

<400> 3
gatatcacca tgcaccacca ccaccaccac gccggaatgt cccccattct cggatactgg 60

aagattaagg gactcgtcca gcccacccgt ctcctcctcg aatacctcga agaaaagtac 120

gaagaacacc tctacgaacg tgacgaagga gacaagtggc gtaacaagaa gttcgaactc 180

ggactcgaat tccccaacct cccctactac attgacggag acgtcaagct cacccagtcc 240

atggccatta ttcgttacat tgccgacaag cacaacatgc tcggaggatg ccccaaggaa 300

cgtgccgaaa tttccatgct cgaaggagcc gtcctcgaca ttcgttacgg agtctcccgt 360

attgcctact ccaaggactt cgaaaccctc aaggtcgact tcctctccaa gctccccgaa 420

atgctcaaga tgttcgaaga ccgtctctgc cacaagacct acctcaacgg agaccacgtc 480

acccaccccg acttcatgct ctacgacgcc ctcgacgtcg tcctctacat ggaccccatg 540

tgcctcgacg ccttccccaa gctcgtctgc ttcaagaagc gtattgaagc cattccccag 600

attgacaagt acctcaagtc ctccaagtac attgcctggc ccctccaggg atggcaggcc 660

accttcggag gaggagacca cccccccaag tccgacggag gacgtgtctc caagggagaa 720

gaactcttca ccggagtcgt ccccattctc gtcgaactcg acggagacgt caacggacac 780

aagttctccg tctccggaga aggagaagga gacgccacct acggaaagct caccctcaag 840

ttcatttgca ccaccggaaa gctccccgtc ccctggccca ccctcgtcac caccctcacc 900

tacggagtcc agtgcttctc ccgttacccc gaccacatga agcagcacga cttcttcaag 960

tccgccatgc ccgaaggata cgtccaggaa cgtaccattt tcttcaagga cgacggaaac 1020

tacaagaccc gtgccgaagt caagttcgaa ggagacaccc tcgtcaaccg tattgaactc 1080

aagggaattg acttcaagga agacggaaac attctcggac acaagctcga atacaactac 1140

aactcccaca acgtctacat tatggccgac aagcagaaga acggaattaa ggtcaacttc 1200

aagattcgtc acaacattga agacggatcc gtccagctcg ccgaccacta ccagcagaac 1260

acccccattg gagacggacc cgtcctcctc cccgacaacc actacctctc cacccagtcc 1320

gccctctcca aggaccccaa cgaaaagcgt gaccacatgg tcctcctcga attcgtcacc 1380

gccgccggaa ttaccctcgg aatggacgaa ctctacaagt ccgacggagg acgtaccgga 1440

gaaaacctct acttccaggg aggacacatg gccgccggat ccattaccac cctccccgcc 1500

ctccccgaag acggaggatc cggagccttc ccccccggac acttcaagga ccccaagcgt 1560

ctctactgca agaacggagg attcttcctc cgtattcacc ccgacggacg tgtcgacgga 1620

gtccgtgaaa agtccgaccc ccacattaag ctccagctcc aggccgaaga acgtggagtc 1680

gtctccatta agggagtctc cgccaaccgt tacctcgcca tgaaggaaga cggacgtctc 1740

ctcgcctcca agtccgtcac cgacgaatgc ttcttcttcg aacgtctcga atccaacaac 1800

tacaacacct accgttcccg taagtacacc tcctggtacg tcgccctcaa gcgtaccgga 1860

cagtacaagc tcggatccaa gaccggaccc ggacagaagg ccattctctt cctccccatg 1920

tccgccaagt cctaatctag a 1941

<210> 4
<211> 1680
<212> DNA
<213> Artificial Sequence

<220>
<223> His-GST-GFP-TEV-IGF1 (HGGTI1)

<400> 4
gatatcacca tgcaccacca ccaccaccac gccggaatgt cccccattct cggatactgg 60

aagattaagg gactcgtcca gcccacccgt ctcctcctcg aatacctcga agaaaagtac 120

gaagaacacc tctacgaacg tgacgaagga gacaagtggc gtaacaagaa gttcgaactc 180

ggactcgaat tccccaacct cccctactac attgacggag acgtcaagct cacccagtcc 240

atggccatta ttcgttacat tgccgacaag cacaacatgc tcggaggatg ccccaaggaa 300

cgtgccgaaa tttccatgct cgaaggagcc gtcctcgaca ttcgttacgg agtctcccgt 360

attgcctact ccaaggactt cgaaaccctc aaggtcgact tcctctccaa gctccccgaa 420

atgctcaaga tgttcgaaga ccgtctctgc cacaagacct acctcaacgg agaccacgtc 480

acccaccccg acttcatgct ctacgacgcc ctcgacgtcg tcctctacat ggaccccatg 540

tgcctcgacg ccttccccaa gctcgtctgc ttcaagaagc gtattgaagc cattccccag 600

attgacaagt acctcaagtc ctccaagtac attgcctggc ccctccaggg atggcaggcc 660

accttcggag gaggagacca cccccccaag tccgacggag gacgtgtctc caagggagaa 720

gaactcttca ccggagtcgt ccccattctc gtcgaactcg acggagacgt caacggacac 780

aagttctccg tctccggaga aggagaagga gacgccacct acggaaagct caccctcaag 840

ttcatttgca ccaccggaaa gctccccgtc ccctggccca ccctcgtcac caccctcacc 900

tacggagtcc agtgcttctc ccgttacccc gaccacatga agcagcacga cttcttcaag 960

tccgccatgc ccgaaggata cgtccaggaa cgtaccattt tcttcaagga cgacggaaac 1020

tacaagaccc gtgccgaagt caagttcgaa ggagacaccc tcgtcaaccg tattgaactc 1080

aagggaattg acttcaagga agacggaaac attctcggac acaagctcga atacaactac 1140

aactcccaca acgtctacat tatggccgac aagcagaaga acggaattaa ggtcaacttc 1200

aagattcgtc acaacattga agacggatcc gtccagctcg ccgaccacta ccagcagaac 1260

acccccattg gagacggacc cgtcctcctc cccgacaacc actacctctc cacccagtcc 1320

gccctctcca aggaccccaa cgaaaagcgt gaccacatgg tcctcctcga attcgtcacc 1380

gccgccggaa ttaccctcgg aatggacgaa ctctacaagt ccgacggagg acgtaccgga 1440

gaaaacctct acttccaggg aggacccgaa accctctgcg gagccgaact cgtcgacgcc 1500

ctccagttcg tctgcggaga ccgtggattc tacttcaaca agcccaccgg atacggatcc 1560

tcctcccgtc gtgcccccca gaccggaatt gtcgacgaat gctgcttccg ttcctgcgac 1620

ctccgtcgtc tcgaaatgta ctgcgccccc ctcaagcccg ccaagtccgc ctaatctaga 1680

1680

<210> 5
<211> 330
<212> DNA
<213> Artificial Sequence

<220>
<223> pPha-HASP1p-HGGTI1

<400> 5
cagaagcgtg ctatcgaact caaccaggga cgtgcggcac aaatgggcat ccttgctctc 60

atggtgcacg aacagttggg agtctctatc cttccttaaa aatttaattt tcattagttg 120

cagtcactcc gctttggttt cacagtcagg aataacacta gctcgtcttc accatggatg 180

ccaatctcgc ctattcatgg tgtataaaag ttcaacatcc aaagctagaa cttttggaaa 240

gagaaagaat atccgaatag ggcacggcgt gccgtattgt tggagtggac tagcagaaag 300

tgaggaaggc acaggatgag ttttctcgag 330

<210> 6
<211> 862
<212> DNA
<213> Artificial Sequence

<220>
<223> Shble cassete

<400> 6
acataccttc agcgtcgtct tcactgtcac agtcaactga cagtaatcgt tgatccggag 60

agattcaaaa ttcaatctgt ttggacctgg ataagacaca agagcgacat cctgacatga 120

acgccgtaaa cagcaaatcc tggttgaaca cgtatccttt tgggggcctc cgctacgacg 180

ctcgctccag ctggggcttc cttactatac acagcgcgca tatttcacgg ttgccagatg 240

tcaagatggc caagttgacc agtgccgttc cggtgctcac cgcgcgcgac gtcgccggag 300

cggtcgagtt ctggaccgac cggctcgggt tctcccggga cttcgtggag gacgacttcg 360

ccggtgtggt ccgggacgac gtgaccctgt tcatcagcgc ggtccaggac caggtggtgc 420

cggacaacac cctggcctgg gtgtgggtgc gcggcctgga cgagctgtac gccgagtggt 480

cggaggtcgt gtccacgaac ttccgggacg cctccgggcc ggccatgacc gagatcggcg 540

agcagccgtg ggggcgggag ttcgccctgc gcgacccggc cggcaactgc gtgcacttcg 600

tggccgagga gcaggactga accttcctta aaaatttaat tttcattagt tgcagtcact 660

ccgctttggt ttcacagtca ggaataacac tagctcgtct tcaccatgga tgccaatctc 720

gcctattcat ggtgtataaa agttcaacat ccaaagctag aacttttgga aagagaaaga 780

atatccgaat agggcacggc gtgccgtatt gttggagtgg actagcagaa agtgaggaag 840

gcacaggatg agttttctcg ag 862

<210> 7
<211> 4059
<212> DNA
<213> Artificial Sequence

<220>
<223> pPha-HASP1p vector full sequence

<400> 7
catatgcata cagtgaatgt aactttcgaa ttgacagtat tagtagtcgt attgacagtg 60

aggcacgccc ctcaatgtgc gaggtggaaa atataccagc atgacaatga atcttggaga 120

ttcttttgct gtcatcaaga ttcaccgcca aatcttcagg aacctatcac gtccacaggc 180

gatgttaatt cttgagtcgt caaaacaaag tcctgtccta cctgtagaag ttgacagcga 240

gcaattgtat gcaaacttct gactttgtta taataacatt aaaggtaatt aagtatcttc 300

aattaggcat tttgtcactg tcagtccgtt ccgacaatat aggtagattt ggaatgaatc 360

ttttctatgc tgctgcgaat cttgtacacc tttgaggccg tagattctgt ccgacgaagc 420

gataattatt gcaaaataca tggactcatt attttgattc gatttctttt tggtatccga 480

ctcgaaaaga tccatcacgg cgagcgaatt cgatatcatc gactaattcg agctcggtac 540

ccggggatcc tctagagtcg acctgcaggc atgcaagctt cagaagcgtg ctatcgaact 600

caaccaggga cgtgcggcac aaatgggcat ccttgctctc atggtgcacg aacagttggg 660

agtctctatc cttccttaaa aatttaattt tcattagttg cagtcactcc gctttggttt 720

cacagtcagg aataacacta gctcgtcttc accatggatg ccaatctcgc ctattcatgg 780

tgtataaaag ttcaacatcc aaagctagaa cttttggaaa gagaaagaat atccgaatag 840

ggcacggcgt gccgtattgt tggagtggac tagcagaaag tgaggaaggc acaggatgag 900

ttttctcgag acataccttc agcgtcgtct tcactgtcac agtcaactga cagtaatcgt 960

tgatccggag agattcaaaa ttcaatctgt ttggacctgg ataagacaca agagcgacat 1020

cctgacatga acgccgtaaa cagcaaatcc tggttgaaca cgtatccttt tgggggcctc 1080

cgctacgacg ctcgctccag ctggggcttc cttactatac acagcgcgca tatttcacgg 1140

ttgccagatg tcaagatggc caagttgacc agtgccgttc cggtgctcac cgcgcgcgac 1200

gtcgccggag cggtcgagtt ctggaccgac cggctcgggt tctcccggga cttcgtggag 1260

gacgacttcg ccggtgtggt ccgggacgac gtgaccctgt tcatcagcgc ggtccaggac 1320

caggtggtgc cggacaacac cctggcctgg gtgtgggtgc gcggcctgga cgagctgtac 1380

gccgagtggt cggaggtcgt gtccacgaac ttccgggacg cctccgggcc ggccatgacc 1440

gagatcggcg agcagccgtg ggggcgggag ttcgccctgc gcgacccggc cggcaactgc 1500

gtgcacttcg tggccgagga gcaggactga accttcctta aaaatttaat tttcattagt 1560

tgcagtcact ccgctttggt ttcacagtca ggaataacac tagctcgtct tcaccatgga 1620

tgccaatctc gcctattcat ggtgtataaa agttcaacat ccaaagctag aacttttgga 1680

aagagaaaga atatccgaat agggcacggc gtgccgtatt gttggagtgg actagcagaa 1740

agtgaggaag gcacaggatg agttttctcg aggccggtct ccctatagtg agtcgtatta 1800

atttcgataa gccaggttaa cctgcattaa tgaatcggcc aacgcgcggg gagaggcggt 1860

ttgcgtattg ggcgctcttc cgcttcctcg ctcactgact cgctgcgctc ggtcgttcgg 1920

ctgcggcgag cggtatcagc tcactcaaag gcggtaatac ggttatccac agaatcaggg 1980

gataacgcag gaaagaacat gtgagcaaaa ggccagcaaa aggccaggaa ccgtaaaaag 2040

gccgcgttgc tggcgttttt ccataggctc cgcccccctg acgagcatca caaaaatcga 2100

cgctcaagtc agaggtggcg aaacccgaca ggactataaa gataccaggc gtttccccct 2160

ggaagctccc tcgtgcgctc tcctgttccg accctgccgc ttaccggata cctgtccgcc 2220

tttctccctt cgggaagcgt ggcgctttct caatgctcac gctgtaggta tctcagttcg 2280

gtgtaggtcg ttcgctccaa gctgggctgt gtgcacgaac cccccgttca gcccgaccgc 2340

tgcgccttat ccggtaacta tcgtcttgag tccaacccgg taagacacga cttatcgcca 2400

ctggcagcag ccactggtaa caggattagc agagcgaggt atgtaggcgg tgctacagag 2460

ttcttgaagt ggtggcctaa ctacggctac actagaagga cagtatttgg tatctgcgct 2520

ctgctgaagc cagttacctt cggaaaaaga gttggtagct cttgatccgg caaacaaacc 2580

accgctggta gcggtggttt ttttgtttgc aagcagcaga ttacgcgcag aaaaaaagga 2640

tctcaagaag atcctttgat cttttctacg gggtctgacg ctcagtggaa cgaaaactca 2700

cgttaaggga ttttggtcat gagattatca aaaaggatct tcacctagat ccttttaaat 2760

taaaaatgaa gttttaaatc aatctaaagt atatatgagt aaacttggtc tgacagttac 2820

caatgcttaa tcagtgaggc acctatctca gcgatctgtc tatttcgttc atccatagtt 2880

gcctgactcc ccgtcgtgta gataactacg atacgggagg gcttaccatc tggccccagt 2940

gctgcaatga taccgcgaga cccacgctca ccggctccag atttatcagc aataaaccag 3000

ccagccggaa gggccgagcg cagaagtggt cctgcaactt tatccgcctc catccagtct 3060

attaattgtt gccgggaagc tagagtaagt agttcgccag ttaatagttt gcgcaacgtt 3120

gttgccattg ctacaggcat cgtggtgtca cgctcgtcgt ttggtatggc ttcattcagc 3180

tccggttccc aacgatcaag gcgagttaca tgatccccca tgttgtgcaa aaaagcggtt 3240

agctccttcg gtcctccgat cgttgtcaga agtaagttgg ccgcagtgtt atcactcatg 3300

gttatggcag cactgcataa ttctcttact gtcatgccat ccgtaagatg cttttctgtg 3360

actggtgagt actcaaccaa gtcattctga gaatagtgta tgcggcgacc gagttgctct 3420

tgcccggcgt caatacggga taataccgcg ccacatagca gaactttaaa agtgctcatc 3480

attggaaaac gttcttcggg gcgaaaactc tcaaggatct taccgctgtt gagatccagt 3540

tcgatgtaac ccactcgtgc acccaactga tcttcagcat cttttacttt caccagcgtt 3600

tctgggtgag caaaaacagg aaggcaaaat gccgcaaaaa agggaataag ggcgacacgg 3660

aaatgttgaa tactcatact cttccttttt caatattatt gaagcattta tcagggttat 3720

tgtctcatga gcggatacat atttgaatgt atttagaaaa ataaacaaat aggggttccg 3780

cgcacatttc cccgaaaagt gccacctgac gtctaagaaa ccattattat catgacatta 3840

acctataaaa ataggcgtat cacgaggccc tttcgtctcg cgcgtttcgg tgatgacggt 3900

gaaaacctct gacacatgca gctcccggag acggtcacag cttgtctgta agcggatgcc 3960

gggagcagac aagcccgtca gggcgcgtca gcgggtgttg gcgggtgtcg gggctggctt 4020

aactatgcgg catcagagca gattgtactg agagtgcac 4059

<210> 8
<211> 5904
<212> DNA
<213> Artificial Sequence

<220>
<223> pPha-HASP1p-HGGTF1

<400> 8
catatgcata cagtgaatgt aactttcgaa ttgacagtat tagtagtcgt attgacagtg 60

aggcacgccc ctcaatgtgc gaggtggaaa atataccagc atgacaatga atcttggaga 120

ttcttttgct gtcatcaaga ttcaccgcca aatcttcagg aacctatcac gtccacaggc 180

gatgttaatt cttgagtcgt caaaacaaag tcctgtccta cctgtagaag ttgacagcga 240

gcaattgtat gcaaacttct gactttgtta taataacatt aaaggtaatt aagtatcttc 300

aattaggcat tttgtcactg tcagtccgtt ccgacaatat aggtagattt ggaatgaatc 360

ttttctatgc tgctgcgaat cttgtacacc tttgaggccg tagattctgt ccgacgaagc 420

gataattatt gcaaaataca tggactcatt attttgattc gatttctttt tggtatccga 480

ctcgaaaaga tccatcacgg cgagcgaatt cgatatcacc atgcaccacc accaccacca 540

cgccggaatg tcccccattc tcggatactg gaagattaag ggactcgtcc agcccacccg 600

tctcctcctc gaatacctcg aagaaaagta cgaagaacac ctctacgaac gtgacgaagg 660

agacaagtgg cgtaacaaga agttcgaact cggactcgaa ttccccaacc tcccctacta 720

cattgacgga gacgtcaagc tcacccagtc catggccatt attcgttaca ttgccgacaa 780

gcacaacatg ctcggaggat gccccaagga acgtgccgaa atttccatgc tcgaaggagc 840

cgtcctcgac attcgttacg gagtctcccg tattgcctac tccaaggact tcgaaaccct 900

caaggtcgac ttcctctcca agctccccga aatgctcaag atgttcgaag accgtctctg 960

ccacaagacc tacctcaacg gagaccacgt cacccacccc gacttcatgc tctacgacgc 1020

cctcgacgtc gtcctctaca tggaccccat gtgcctcgac gccttcccca agctcgtctg 1080

cttcaagaag cgtattgaag ccattcccca gattgacaag tacctcaagt cctccaagta 1140

cattgcctgg cccctccagg gatggcaggc caccttcgga ggaggagacc acccccccaa 1200

gtccgacgga ggacgtgtct ccaaggagaa agaactcttc accggagtcg tccccattct 1260

cgtcgaactc gacggagacg tcaacggaca caagttctcc gtctccggag aaggagaagg 1320

agacgccacc tacggaaagc tcaccctcaa gttcatttgc accaccggaa agctccccgt 1380

cccctggccc accctcgtca ccaccctcac ctacggagtc cagtgcttct cccgttaccc 1440

cgaccacatg aagcagcacg acttcttcaa gtccgccatg cccgaaggat acgtccagga 1500

acgtaccatt ttcttcaagg acgacggaaa ctacaagacc cgtgccgaag tcaagttcga 1560

aggagacacc ctcgtcaacc gtattgaact caagggaatt gacttcaagg aagacggaaa 1620

cattctcgga cacaagctcg aatacaacta caactcccac aacgtctaca ttatggccga 1680

caagcagaag aacggaatta aggtcaactt caagattcgt cacaacattg aagacggatc 1740

cgtccagctc gccgaccact accagcagaa cacccccatt ggagacggac ccgtcctcct 1800

ccccgacaac cactacctct ccacccagtc cgccctctcc aaggacccca acgaaaagcg 1860

tgaccacatg gtcctcctcg aattcgtcac cgccgccgga attaccctcg gaatggacga 1920

actctacaag tccgacggag gacgtaccgg agaaaacctc tacttccagg gattcaacct 1980

cccccccgga aactacaaga agcccaagct cctctactgc tccaacggag gacacttcct 2040

ccgtattctc cccgacggaa ccgtcgacgg aacccgtgac cgttccgacc agcacattca 2100

gctccagctc tccgccgaat ccgtcggaga agtctacatt aagtccaccg aaaccggaca 2160

gtacctcgcc atggacaccg acggactcct ctacggatcc cagaccccca acgaagaatg 2220

cctcttcctc gaacgtctcg aagaaaacca ctacaacacc tacatttcca agaagcacgc 2280

cgaaaagaac tggttcgtcg gactcaagaa gaacggatcc tgcaagcgtg gaccccgtac 2340

ccactacgga cagaaggcca ttctcttcct cccccctccc gtctcctccg actaatctag 2400

agtcgacctg caggcatgca agcttcagaa gcgtgctatc gaactcaacc agggacgtgc 2460

ggcacaaatg ggcatccttg ctctcatggt gcacgaacag ttgggagtct ctatccttcc 2520

ttaaaaattt aattttcatt agttgcagtc actccgcttt ggtttcacag tcaggaataa 2580

cactagctcg tcttcaccat ggatgccaat ctcgcctatt catggtgtat aaaagttcaa 2640

catccaaagc tagaactttt ggaaagagaa agaatatccg aatagggcac ggcgtgccgt 2700

attgttggag tggactagca gaaagtgagg aaggcacagg atgagttttc tcgagacata 2760

ccttcagcgt cgtcttcact gtcacagtca actgacagta atcgttgatc cggagagatt 2820

caaaattcaa tctgtttgga cctggataag acacaagagc gacatcctga catgaacgcc 2880

gtaaacagca aatcctggtt gaacacgtat ccttttgggg gcctccgcta cgacgctcgc 2940

tccagctggg gcttccttac tatacacagc gcgcatattt cacggttgcc agatgtcaag 3000

atggccaagt tgaccagtgc cgttccggtg ctcaccgcgc gcgacgtcgc cggagcggtc 3060

gagttctgga ccgaccggct cgggttctcc cgggacttcg tggaggacga cttcgccggt 3120

gtggtccggg acgacgtgac cctgttcatc agcgcggtcc aggaccaggt ggtgccggac 3180

aacaccctgg cctgggtgtg ggtgcgcggc ctggacgagc tgtacgccga gtggtcggag 3240

gtcgtgtcca cgaacttccg ggacgcctcc gggccggcca tgaccgagat cggcgagcag 3300

ccgtgggggc gggagttcgc cctgcgcgac ccggccggca actgcgtgca cttcgtggcc 3360

gaggagcagg actgaacctt ccttaaaaat ttaattttca ttagttgcag tcactccgct 3420

ttggtttcac agtcaggaat aacactagct cgtcttcacc atggatgcca atctcgccta 3480

ttcatggtgt ataaaagttc aacatccaaa gctagaactt ttggaaagag aaagaatatc 3540

cgaatagggc acggcgtgcc gtattgttgg agtggactag cagaaagtga ggaaggcaca 3600

ggatgagttt tctcgaggcc ggtctcccta tagtgagtcg tattaatttc gataagccag 3660

gttaacctgc attaatgaat cggccaacgc gcggggagag gcggtttgcg tattgggcgc 3720

tcttccgctt cctcgctcac tgactcgctg cgctcggtcg ttcggctgcg gcgagcggta 3780

tcagctcact caaaggcggt aatacggtta tccacagaat caggggataa cgcaggaaag 3840

aacatgtgag caaaaggcca gcaaaaggcc aggaaccgta aaaaggccgc gttgctggcg 3900

tttttccata ggctccgccc ccctgacgag catcacaaaa atcgacgctc aagtcagagg 3960

tggcgaaacc cgacaggact ataaagatac caggcgtttc cccctggaag ctccctcgtg 4020

cgctctcctg ttccgaccct gccgcttacc ggatacctgt ccgcctttct cccttcggga 4080

agcgtggcgc tttctcaatg ctcacgctgt aggtatctca gttcggtgta ggtcgttcgc 4140

tccaagctgg gctgtgtgca cgaacccccc gttcagcccg accgctgcgc cttatccggt 4200

aactatcgtc ttgagtccaa cccggtaaga cacgacttat cgccactggc agcagccact 4260

ggtaacagga ttagcagagc gaggtatgta ggcggtgcta cagagttctt gaagtggtgg 4320

cctaactacg gctacactag aaggacagta tttggtatct gcgctctgct gaagccagtt 4380

accttcggaa aaagagttgg tagctcttga tccggcaaac aaaccaccgc tggtagcggt 4440

ggtttttttg tttgcaagca gcagattacg cgcagaaaaa aaggatctca agaagatcct 4500

ttgatctttt ctacggggtc tgacgctcag tggaacgaaa actcacgtta agggattttg 4560

gtcatgagat tatcaaaaag gatcttcacc tagatccttt taaattaaaa atgaagtttt 4620

aaatcaatct aaagtatata tgagtaaact tggtctgaca gttaccaatg cttaatcagt 4680

gaggcaccta tctcagcgat ctgtctattt cgttcatcca tagttgcctg actccccgtc 4740

gtgtagataa ctacgatacg ggagggctta ccatctggcc ccagtgctgc aatgataccg 4800

cgagacccac gctcaccggc tccagattta tcagcaataa accagccagc cggaagggcc 4860

gagcgcagaa gtggtcctgc aactttatcc gcctccatcc agtctattaa ttgttgccgg 4920

gaagctagag taagtagttc gccagttaat agtttgcgca acgttgttgc cattgctaca 4980

ggcatcgtgg tgtcacgctc gtcgtttggt atggcttcat tcagctccgg ttcccaacga 5040

tcaaggcgag ttacatgatc ccccatgttg tgcaaaaaag cggttagctc cttcggtcct 5100

ccgatcgttg tcagaagtaa gttggccgca gtgttatcac tcatggttat ggcagcactg 5160

cataattctc ttactgtcat gccatccgta agatgctttt ctgtgactgg tgagtactca 5220

accaagtcat tctgagaata gtgtatgcgg cgaccgagtt gctcttgccc ggcgtcaata 5280

cgggataata ccgcgccaca tagcagaact ttaaaagtgc tcatcattgg aaaacgttct 5340

tcggggcgaa aactctcaag gatcttaccg ctgttgagat ccagttcgat gtaacccact 5400

cgtgcaccca actgatcttc agcatctttt actttcacca gcgtttctgg gtgagcaaaa 5460

acaggaaggc aaaatgccgc aaaaaaggga ataagggcga cacggaaatg ttgaatactc 5520

atactcttcc tttttcaata ttattgaagc atttatcagg gttattgtct catgagcgga 5580

tacatatttg aatgtattta gaaaaataaa caaatagggg ttccgcgcac atttccccga 5640

aaagtgccac ctgacgtcta agaaaccatt attatcatga cattaaccta taaaaatagg 5700

cgtatcacga ggccctttcg tctcgcgcgt ttcggtgatg acggtgaaaa cctctgacac 5760

atgcagctcc cggagacggt cacagcttgt ctgtaagcgg atgccgggag cagacaagcc 5820

cgtcagggcg cgtcagcggg tgttggcggg tgtcggggct ggcttaacta tgcggcatca 5880

gagcagattg tactgagagt gcac 5904

<210> 9
<211> 5955
<212> DNA
<213> Artificial Sequence

<220>
<223> pPha-HASP1p-HGGTF2

<400> 9

catatgcata cagtgaatgt aactttcgaa ttgacagtat tagtagtcgt attgacagtg 60

aggcacgccc ctcaatgtgc gaggtggaaa atataccagc atgacaatga atcttggaga 120

ttcttttgct gtcatcaaga ttcaccgcca aatcttcagg aacctatcac gtccacaggc 180

gatgttaatt cttgagtcgt caaaacaaag tcctgtccta cctgtagaag ttgacagcga 240

gcaattgtat gcaaacttct gactttgtta taataacatt aaaggtaatt aagtatcttc 300

aattaggcat tttgtcactg tcagtccgtt ccgacaatat aggtagattt ggaatgaatc 360

ttttctatgc tgctgcgaat cttgtacacc tttgaggccg tagattctgt ccgacgaagc 420

gataattatt gcaaaataca tggactcatt attttgattc gatttctttt tggtatccga 480

ctcgaaaaga tccatcacgg cgagcgaatt cgatatcacc atgcaccacc accaccacca 540

cgccggaatg tcccccattc tcggatactg gaagattaag ggactcgtcc agcccacccg 600

tctcctcctc gaatacctcg aagaaaagta cgaagaacac ctctacgaac gtgacgaagg 660

agacaagtgg cgtaacaaga agttcgaact cggactcgaa ttccccaacc tcccctacta 720

cattgacgga gacgtcaagc tcacccagtc catggccatt attcgttaca ttgccgacaa 780

gcacaacatg ctcggaggat gccccaagga acgtgccgaa atttccatgc tcgaaggagc 840

cgtcctcgac attcgttacg gagtctcccg tattgcctac tccaaggact tcgaaaccct 900

caaggtcgac ttcctctcca agctccccga aatgctcaag atgttcgaag accgtctctg 960

ccacaagacc tacctcaacg gagaccacgt cacccacccc gacttcatgc tctacgacgc 1020

cctcgacgtc gtcctctaca tggaccccat gtgcctcgac gccttcccca agctcgtctg 1080

cttcaagaag cgtattgaag ccattcccca gattgacaag tacctcaagt cctccaagta 1140

cattgcctgg cccctccagg gatggcaggc caccttcgga ggaggagacc acccccccaa 1200

gtccgacgga ggacgtgtct ccaaggagaa agaactcttc accggagtcg tccccattct 1260

cgtcgaactc gacggagacg tcaacggaca caagttctcc gtctccggag aaggagaagg 1320

agacgccacc tacggaaagc tcaccctcaa gttcatttgc accaccggaa agctccccgt 1380

cccctggccc accctcgtca ccaccctcac ctacggagtc cagtgcttct cccgttaccc 1440

cgaccacatg aagcagcacg acttcttcaa gtccgccatg cccgaaggat acgtccagga 1500

acgtaccatt ttcttcaagg acgacggaaa ctacaagacc cgtgccgaag tcaagttcga 1560

aggagacacc ctcgtcaacc gtattgaact caagggaatt gacttcaagg aagacggaaa 1620

cattctcgga cacaagctcg aatacaacta caactcccac aacgtctaca ttatggccga 1680

caagcagaag aacggaatta aggtcaactt caagattcgt cacaacattg aagacggatc 1740

cgtccagctc gccgaccact accagcagaa cacccccatt ggagacggac ccgtcctcct 1800

ccccgacaac cactacctct ccacccagtc cgccctctcc aaggacccca acgaaaagcg 1860

tgaccacatg gtcctcctcg aattcgtcac cgccgccgga attaccctcg gaatggacga 1920

actctacaag tccgacggag gacgtaccgg agaaaacctc tacttccagg gaggacacat 1980

ggccgccgga tccattacca ccctccccgc cctccccgaa gacggaggat ccggagcctt 2040

cccccccgga cacttcaagg accccaagcg tctctactgc aagaacggag gattcttcct 2100

ccgtattcac cccgacggac gtgtcgacgg agtccgtgaa aagtccgacc cccacattaa 2160

gctccagctc caggccgaag aacgtggagt cgtctccatt aagggagtct ccgccaaccg 2220

ttacctcgcc atgaaggaag acggacgtct cctcgcctcc aagtccgtca ccgacgaatg 2280

cttcttcttc gaacgtctcg aatccaacaa ctacaacacc taccgttccc gtaagtacac 2340

ctcctggtac gtcgccctca agcgtaccgg acagtacaag ctcggatcca agaccggacc 2400

cggacagaag gccattctct tcctccccat gtccgccaag tcctaatcta gagtcgacct 2460

gcaggcatgc aagcttcaga agcgtgctat cgaactcaac cagggacgtg cggcacaaat 2520

gggcatcctt gctctcatgg tgcacgaaca gttgggagtc tctatccttc cttaaaaatt 2580

taattttcat tagttgcagt cactccgctt tggtttcaca gtcaggaata acactagctc 2640

gtcttcacca tggatgccaa tctcgcctat tcatggtgta taaaagttca acatccaaag 2700

ctagaacttt tggaaagaga aagaatatcc gaatagggca cggcgtgccg tattgttgga 2760

gtggactagc agaaagtgag gaaggcacag gatgagtttt ctcgagacat accttcagcg 2820

tcgtcttcac tgtcacagtc aactgacagt aatcgttgat ccggagagat tcaaaattca 2880

atctgtttgg acctggataa gacacaagag cgacatcctg acatgaacgc cgtaaacagc 2940

aaatcctggt tgaacacgta tccttttggg ggcctccgct acgacgctcg ctccagctgg 3000

ggcttcctta ctatacacag cgcgcatatt tcacggttgc cagatgtcaa gatggccaag 3060

ttgaccagtg ccgttccggt gctcaccgcg cgcgacgtcg ccggagcggt cgagttctgg 3120

accgaccggc tcgggttctc ccgggacttc gtggaggacg acttcgccgg tgtggtccgg 3180

gacgacgtga ccctgttcat cagcgcggtc caggaccagg tggtgccgga caacaccctg 3240

gcctgggtgt gggtgcgcgg cctggacgag ctgtacgccg agtggtcgga ggtcgtgtcc 3300

acgaacttcc gggacgcctc cgggccggcc atgaccgaga tcggcgagca gccgtggggg 3360

cgggagttcg ccctgcgcga cccggccggc aactgcgtgc acttcgtggc cgaggagcag 3420

gactgaacct tccttaaaaa tttaattttc attagttgca gtcactccgc tttggtttca 3480

cagtcaggaa taacactagc tcgtcttcac catggatgcc aatctcgcct attcatggtg 3540

tataaaagtt caacatccaa agctagaact tttggaaaga gaaagaatat ccgaataggg 3600

cacggcgtgc cgtattgttg gagtggacta gcagaaagtg aggaaggcac aggatgagtt 3660

ttctcgaggc cggtctccct atagtgagtc gtattaattt cgataagcca ggttaacctg 3720

cattaatgaa tcggccaacg cgcggggaga ggcggtttgc gtattgggcg ctcttccgct 3780

tcctcgctca ctgactcgct gcgctcggtc gttcggctgc ggcgagcggt atcagctcac 3840

tcaaaggcgg taatacggtt atccacagaa tcaggggata acgcaggaaa gaacatgtga 3900

gcaaaaggcc agcaaaaggc caggaaccgt aaaaaggccg cgttgctggc gtttttccat 3960

aggctccgcc cccctgacga gcatcacaaa aatcgacgct caagtcagag gtggcgaaac 4020

ccgacaggac tataaagata ccaggcgttt ccccctggaa gctccctcgt gcgctctcct 4080

gttccgaccc tgccgcttac cggatacctg tccgcctttc tcccttcggg aagcgtggcg 4140

ctttctcaat gctcacgctg taggtatctc agttcggtgt aggtcgttcg ctccaagctg 4200

ggctgtgtgc acgaaccccc cgttcagccc gaccgctgcg ccttatccgg taactatcgt 4260

cttgagtcca acccggtaag acacgactta tcgccactgg cagcagccac tggtaacagg 4320

attagcagag cgaggtatgt aggcggtgct acagagttct tgaagtggtg gcctaactac 4380

ggctacacta gaaggacagt atttggtatc tgcgctctgc tgaagccagt taccttcgga 4440

aaaagagttg gtagctcttg atccggcaaa caaaccaccg ctggtagcgg tggttttttt 4500

gtttgcaagc agcagattac gcgcagaaaa aaaggatctc aagaagatcc tttgatcttt 4560

tctacggggt ctgacgctca gtggaacgaa aactcacgtt aagggatttt ggtcatgaga 4620

ttatcaaaaa ggatcttcac ctagatcctt ttaaattaaa aatgaagttt taaatcaatc 4680

taaagtatat atgagtaaac ttggtctgac agttaccaat gcttaatcag tgaggcacct 4740

atctcagcga tctgtctatt tcgttcatcc atagttgcct gactccccgt cgtgtagata 4800

actacgatac gggagggctt accatctggc cccagtgctg caatgatacc gcgagaccca 4860

cgctcaccgg ctccagattt atcagcaata aaccagccag ccggaagggc cgagcgcaga 4920

agtggtcctg caactttatc cgcctccatc cagtctatta attgttgccg ggaagctaga 4980

gtaagtagtt cgccagttaa tagtttgcgc aacgttgttg ccattgctac aggcatcgtg 5040

gtgtcacgct cgtcgtttgg tatggcttca ttcagctccg gttcccaacg atcaaggcga 5100

gttacatgat cccccatgtt gtgcaaaaaa gcggttagct ccttcggtcc tccgatcgtt 5160

gtcagaagta agttggccgc agtgttatca ctcatggtta tggcagcact gcataattct 5220

cttactgtca tgccatccgt aagatgcttt tctgtgactg gtgagtactc aaccaagtca 5280

ttctgagaat agtgtatgcg gcgaccgagt tgctcttgcc cggcgtcaat acgggataat 5340

accgcgccac atagcagaac tttaaaagtg ctcatcattg gaaaacgttc ttcggggcga 5400

aaactctcaa ggatcttacc gctgttgaga tccagttcga tgtaacccac tcgtgcaccc 5460

aactgatctt cagcatcttt tactttcacc agcgtttctg ggtgagcaaa aacaggaagg 5520

caaaatgccg caaaaaaggg aataagggcg acacggaaat gttgaatact catactcttc 5580

ctttttcaat attattgaag catttatcag ggttattgtc tcatgagcgg atacatattt 5640

gaatgtattt agaaaaataa acaaataggg gttccgcgca catttccccg aaaagtgcca 5700

cctgacgtct aagaaaccat tattatcatg acattaacct ataaaaatag gcgtatcacg 5760

aggccctttc gtctcgcgcg tttcggtgat gacggtgaaa acctctgaca catgcagctc 5820

ccggagacgg tcacagcttg tctgtaagcg gatgccggga gcagacaagc ccgtcagggc 5880

gcgtcagcgg gtgttggcgg gtgtcggggc tggcttaact atgcggcatc agagcagatt 5940

gtactgagag tgcac 5955

<210> 10
<211> 5694
<212> DNA
<213> Artificial Sequence

<220>
<223> pPha-HASP1p-HGGTI1

<400> 10
catatgcata cagtgaatgt aactttcgaa ttgacagtat tagtagtcgt attgacagtg 60

aggcacgccc ctcaatgtgc gaggtggaaa atataccagc atgacaatga atcttggaga 120

ttcttttgct gtcatcaaga ttcaccgcca aatcttcagg aacctatcac gtccacaggc 180

gatgttaatt cttgagtcgt caaaacaaag tcctgtccta cctgtagaag ttgacagcga 240

gcaattgtat gcaaacttct gactttgtta taataacatt aaaggtaatt aagtatcttc 300

aattaggcat tttgtcactg tcagtccgtt ccgacaatat aggtagattt ggaatgaatc 360

ttttctatgc tgctgcgaat cttgtacacc tttgaggccg tagattctgt ccgacgaagc 420

gataattatt gcaaaataca tggactcatt attttgattc gatttctttt tggtatccga 480

ctcgaaaaga tccatcacgg cgagcgaatt cgatatcacc atgcaccacc accaccacca 540

cgccggaatg tcccccattc tcggatactg gaagattaag ggactcgtcc agcccacccg 600

tctcctcctc gaatacctcg aagaaaagta cgaagaacac ctctacgaac gtgacgaagg 660

agacaagtgg cgtaacaaga agttcgaact cggactcgaa ttccccaacc tcccctacta 720

cattgacgga gacgtcaagc tcacccagtc catggccatt attcgttaca ttgccgacaa 780

gcacaacatg ctcggaggat gccccaagga acgtgccgaa atttccatgc tcgaaggagc 840

cgtcctcgac attcgttacg gagtctcccg tattgcctac tccaaggact tcgaaaccct 900

caaggtcgac ttcctctcca agctccccga aatgctcaag atgttcgaag accgtctctg 960

ccacaagacc tacctcaacg gagaccacgt cacccacccc gacttcatgc tctacgacgc 1020

cctcgacgtc gtcctctaca tggaccccat gtgcctcgac gccttcccca agctcgtctg 1080

cttcaagaag cgtattgaag ccattcccca gattgacaag tacctcaagt cctccaagta 1140

cattgcctgg cccctccagg gatggcaggc caccttcgga ggaggagacc acccccccaa 1200

gtccgacgga ggacgtgtct ccaaggagaa agaactcttc accggagtcg tccccattct 1260

cgtcgaactc gacggagacg tcaacggaca caagttctcc gtctccggag aaggagaagg 1320

agacgccacc tacggaaagc tcaccctcaa gttcatttgc accaccggaa agctccccgt 1380

cccctggccc accctcgtca ccaccctcac ctacggagtc cagtgcttct cccgttaccc 1440

cgaccacatg aagcagcacg acttcttcaa gtccgccatg cccgaaggat acgtccagga 1500

acgtaccatt ttcttcaagg acgacggaaa ctacaagacc cgtgccgaag tcaagttcga 1560

aggagacacc ctcgtcaacc gtattgaact caagggaatt gacttcaagg aagacggaaa 1620

cattctcgga cacaagctcg aatacaacta caactcccac aacgtctaca ttatggccga 1680

caagcagaag aacggaatta aggtcaactt caagattcgt cacaacattg aagacggatc 1740

cgtccagctc gccgaccact accagcagaa cacccccatt ggagacggac ccgtcctcct 1800

ccccgacaac cactacctct ccacccagtc cgccctctcc aaggacccca acgaaaagcg 1860

tgaccacatg gtcctcctcg aattcgtcac cgccgccgga attaccctcg gaatggacga 1920

actctacaag tccgacggag gacgtaccgg agaaaacctc tacttccagg gaggacccga 1980

aaccctctgc ggagccgaac tcgtcgacgc cctccagttc gtctgcggag accgtggatt 2040

ctacttcaac aagcccaccg gatacggatc ctcctcccgt cgtgcccccc agaccggaat 2100

tgtcgacgaa tgctgcttcc gttcctgcga cctccgtcgt ctcgaaatgt actgcgcccc 2160

cctcaagccc gccaagtccg cctaatctag agtcgacctg caggcatgca agcttcagaa 2220

gcgtgctatc gaactcaacc agggacgtgc ggcacaaatg ggcatccttg ctctcatggt 2280

gcacgaacag ttgggagtct ctatccttcc ttaaaaattt aattttcatt agttgcagtc 2340

actccgcttt ggtttcacag tcaggaataa cactagctcg tcttcaccat ggatgccaat 2400

ctcgcctatt catggtgtat aaaagttcaa catccaaagc tagaactttt ggaaagagaa 2460

agaatatccg aatagggcac ggcgtgccgt attgttggag tggactagca gaaagtgagg 2520

aaggcacagg atgagttttc tcgagacata ccttcagcgt cgtcttcact gtcacagtca 2580

actgacagta atcgttgatc cggagagatt caaaattcaa tctgtttgga cctggataag 2640

acacaagagc gacatcctga catgaacgcc gtaaacagca aatcctggtt gaacacgtat 2700

ccttttgggg gcctccgcta cgacgctcgc tccagctggg gcttccttac tatacacagc 2760

gcgcatattt cacggttgcc agatgtcaag atggccaagt tgaccagtgc cgttccggtg 2820

ctcaccgcgc gcgacgtcgc cggagcggtc gagttctgga ccgaccggct cgggttctcc 2880

cgggacttcg tggaggacga cttcgccggt gtggtccggg acgacgtgac cctgttcatc 2940

agcgcggtcc aggaccaggt ggtgccggac aacaccctgg cctgggtgtg ggtgcgcggc 3000

ctggacgagc tgtacgccga gtggtcggag gtcgtgtcca cgaacttccg ggacgcctcc 3060

gggccggcca tgaccgagat cggcgagcag ccgtgggggc gggagttcgc cctgcgcgac 3120

ccggccggca actgcgtgca cttcgtggcc gaggagcagg actgaacctt ccttaaaaat 3180

ttaattttca ttagttgcag tcactccgct ttggtttcac agtcaggaat aacactagct 3240

cgtcttcacc atggatgcca atctcgccta ttcatggtgt ataaaagttc aacatccaaa 3300

gctagaactt ttggaaagag aaagaatatc cgaatagggc acggcgtgcc gtattgttgg 3360

agtggactag cagaaagtga ggaaggcaca ggatgagttt tctcgaggcc ggtctcccta 3420

tagtgagtcg tattaatttc gataagccag gttaacctgc attaatgaat cggccaacgc 3480

gcggggagag gcggtttgcg tattgggcgc tcttccgctt cctcgctcac tgactcgctg 3540

cgctcggtcg ttcggctgcg gcgagcggta tcagctcact caaaggcggt aatacggtta 3600

tccacagaat caggggataa cgcaggaaag aacatgtgag caaaaggcca gcaaaaggcc 3660

aggaaccgta aaaaggccgc gttgctggcg tttttccata ggctccgccc ccctgacgag 3720

catcacaaaa atcgacgctc aagtcagagg tggcgaaacc cgacaggact ataaagatac 3780

caggcgtttc cccctggaag ctccctcgtg cgctctcctg ttccgaccct gccgcttacc 3840

ggatacctgt ccgcctttct cccttcggga agcgtggcgc tttctcaatg ctcacgctgt 3900

aggtatctca gttcggtgta ggtcgttcgc tccaagctgg gctgtgtgca cgaacccccc 3960

gttcagcccg accgctgcgc cttatccggt aactatcgtc ttgagtccaa cccggtaaga 4020

cacgacttat cgccactggc agcagccact ggtaacagga ttagcagagc gaggtatgta 4080

ggcggtgcta cagagttctt gaagtggtgg cctaactacg gctacactag aaggacagta 4140

tttggtatct gcgctctgct gaagccagtt accttcggaa aaagagttgg tagctcttga 4200

tccggcaaac aaaccaccgc tggtagcggt ggtttttttg tttgcaagca gcagattacg 4260

cgcagaaaaa aaggatctca agaagatcct ttgatctttt ctacggggtc tgacgctcag 4320

tggaacgaaa actcacgtta agggattttg gtcatgagat tatcaaaaag gatcttcacc 4380

tagatccttt taaattaaaa atgaagtttt aaatcaatct aaagtatata tgagtaaact 4440

tggtctgaca gttaccaatg cttaatcagt gaggcaccta tctcagcgat ctgtctattt 4500

cgttcatcca tagttgcctg actccccgtc gtgtagataa ctacgatacg ggagggctta 4560

ccatctggcc ccagtgctgc aatgataccg cgagacccac gctcaccggc tccagattta 4620

tcagcaataa accagccagc cggaagggcc gagcgcagaa gtggtcctgc aactttatcc 4680

gcctccatcc agtctattaa ttgttgccgg gaagctagag taagtagttc gccagttaat 4740

agtttgcgca acgttgttgc cattgctaca ggcatcgtgg tgtcacgctc gtcgtttggt 4800

atggcttcat tcagctccgg ttcccaacga tcaaggcgag ttacatgatc ccccatgttg 4860

tgcaaaaaag cggttagctc cttcggtcct ccgatcgttg tcagaagtaa gttggccgca 4920

gtgttatcac tcatggttat ggcagcactg cataattctc ttactgtcat gccatccgta 4980

agatgctttt ctgtgactgg tgagtactca accaagtcat tctgagaata gtgtatgcgg 5040

cgaccgagtt gctcttgccc ggcgtcaata cgggataata ccgcgccaca tagcagaact 5100

ttaaaagtgc tcatcattgg aaaacgttct tcggggcgaa aactctcaag gatcttaccg 5160

ctgttgagat ccagttcgat gtaacccact cgtgcaccca actgatcttc agcatctttt 5220

actttcacca gcgtttctgg gtgagcaaaa acaggaaggc aaaatgccgc aaaaaaggga 5280

ataagggcga cacggaaatg ttgaatactc atactcttcc tttttcaata ttattgaagc 5340

atttatcagg gttattgtct catgagcgga tacatatttg aatgtattta gaaaaataaa 5400

caaatagggg ttccgcgcac atttccccga aaagtgccac ctgacgtcta agaaaccatt 5460

attatcatga cattaaccta taaaaatagg cgtatcacga ggccctttcg tctcgcgcgt 5520

ttcggtgatg acggtgaaaa cctctgacac atgcagctcc cggagacggt cacagcttgt 5580

ctgtaagcgg atgccgggag cagacaagcc cgtcagggcg cgtcagcggg tgttggcggg 5640

tgtcggggct ggcttaacta tgcggcatca gagcagattg tactgagagt gcac 5694